# EUROPEAN PATENT APPLICATION

(11) **EP 4 353 252 A1**
(43) Date of publication of application: **17.04.2024**
(21) Application number: 22200487.1
(22) Date of filing: 10.10.2022
(51) Int. Cl.: A61K 39/00, C07K 14/725, C07K 16/28, C12N 5/0783, C12N 15/113

(54) **ANTIGEN-SPECIFIC T CELLS BY GENE EDITING OF CD3 EPSILON**

(71) Applicant: Charité - Universitätsmedizin Berlin, 10117 Berlin (DE)
(72) Inventor: VOLK, Hans-Dieter, 10117 Berlin (DE); REINKE, Petra, 10117 Berlin (DE); SCHMÜCK-HENNERESSE, Michael, 10247 Berlin (DE); WAGNER, Dimitrios Laurin, 13347 Berlin (DE); KATH, Jonas Christian, 10553 Berlin (DE); DU, Weijie, 16321 Bernau (DE)
(74) Representative: Hertin und Partner Rechts- und Patentanwälte PartG mbB

(57) **Abstract**

The invention relates to a nucleic acid construct for targeting and integrating a binding domain in-frame into an endogenous CD3 epsilon gene of a human cell. The invention further relates to a genetically modified human T cell comprising an exogenous nucleic acid sequence region encoding an antigen binding domain integrated in-frame into an endogenous CD3 epsilon gene. In preferred embodiments the T cell is a regulatory T cell (Treg). The invention further relates to the genetically modified T cell for use as a medicament for the prevention and/or treatment of an unwanted (pathogenic) immune reaction, preferably an unwanted (pathogenic) immune reaction prior to and/or after an allogeneic transplantation or an autoimmune disease, more preferably for the prevention and/or treatment of graft-versus-host disease (GVHD). The invention further relates to a method for preparing a therapeutic genetically modified T cell, comprising repetitive stimulation via the TCR/CD3 complex and CD28 co-receptor, wherein said therapeutic genetically modified regulatory T cell exhibits in essence an unmodified expansion in culture (+/- 50%) in comparison to an unmodified T cell.

## Description

The invention relates to the field of biology and medicine, in particular genetic modification of therapeutic cell products.

The invention relates to a nucleic acid construct for targeting and integrating a binding domain in-frame into an endogenous CD3 epsilon gene to generate a gene fusion and therefore a modified functional CD3-T cell receptor (TCR) complex comprising an exogenous binding domain, such as an antibody or antigen-binding fragment thereof, fused with an endogenous CD3 epsilon protein.

The invention further relates to a nucleic acid construct, comprising two nucleic acid sequence regions, wherein a first nucleic acid sequence region encodes an antigen binding domain, and a second nucleic acid sequence region encodes a targeting sequence configured for integrating the construct in-frame with an endogenous CD3 epsilon gene of a human cell.

In further aspects, the invention relates to a genetically modified human T cell, comprising an exogenous nucleic acid sequence region encoding an antigen binding domain integrated in-frame into an endogenous CD3 epsilon gene. The invention further relates to a genetically modified human T cell expressing a CD3 epsilon protein as an in-frame fusion protein comprising the antigen binding domain and endogenous CD3 epsilon protein, wherein the T cell receptor (TCR) function is maintained in the presence of the in-frame fusion protein. The modified T cell is preferably a regulatory T cell (Treg). The invention further relates to a polypeptide expressed from the genetically modified T cell as a fusion protein comprising an antigen binding domain and the endogenous CD3 epsilon protein.

The invention further relates to a pharmaceutical composition comprising a genetically modified T cell, preferably a Treg, and a pharmaceutically acceptable carrier for therapeutic interventions in human diseases. The invention further relates to corresponding medical uses and therapeutic methods in the treatment and/or prevention of a medical condition, such as an unwanted (pathogenic) immune reaction, including an unwanted (pathogenic) immune reaction after an allogenic transplantation, and for the prevention and/or treatment of graft-versus-host disease (GvHD).

In further aspects the invention relates to a method for preparing a therapeutic genetically modified T cell, preferably a Treg cell, comprising repetitive stimulation of a genetically modified T cell expressing a modified functional CD3-TCR complex with an in-frame fusion protein comprising an exogenous antigen binding domain and endogenous CD3 epsilon protein, wherein said therapeutic genetically modified T cell, preferably genetically modified Treg cell, exhibits in essence unmodified expansion dynamics in culture (+/- 50%) in comparison to an unmodified T cell.

The invention further relates to oligonucleotides, such as gRNAs and primers, suitable for the inventive in-frame integration of a nucleic acid construct into an endogenous CD3 epsilon gene or amplification of the nucleic acid construct.

### BACKGROUND OF THE INVENTION

Adoptive transfer of immune cells is an immunotherapeutic treatment with great potential for the treatment of immune-mediated diseases and of cancer. In particular, adoptive transfer of T cells, such as regulatory T cells (Treg), has become of significant interest. Treg cells are immunosuppressive lymphocytes that prevent autoimmune diseases, modulate and end overshooting immune responses, ensure tissue regeneration and regulate bacterial homeostasis at mucosal surfaces. Clinical data from patients with solid organ or hematopoietic stem cell transplantation further associate a higher frequency of activated Treg cells after transplantation with better graft survival and lower incidence of graft-versus-host-disease (GvHD). Congenital Treg cell dysfunction is associated with severe autoimmune disease. Consequently, adoptive transfer of Treg cells is an emerging modality for tailored immunosuppression with many potential applications.

Adoptive transfer of ex vivo expanded polyclonal Treg cells has been clinically tested as a potential treatment to induce tolerance toward solid organ transplants (Roemhild et al., 2020), resolve chronic GvHD (Landwehr-Kenzel et al., 2022), ameliorate autoimmune diseases, such as type 1 diabetes mellitus (Marek-Trzonkowska et al., 2014) or inflammatory bowel diseases (Voskens et al., 2022). Transfer of antigen-specific Treg cells demonstrates high potency in most preclinical models of above-mentioned diseases (Amini et al., 2021) due to superior homing and tissue-specific activation. In a clinical study with kidney transplant patients, it was shown that the transfer of autologous, ex vivo expanded Treg cells allows a reduction of drug immunosuppression without leading to increased graft rejection. In summary, the study demonstrates that Treg cell therapy is safe and does not provoke increased infections, and further can efficiently replace drug immunosuppression (Roemhild et al., 2020). A further study showed that adoptive transfer of donor Tregs cells after allogeneic stem cell transplantation in refractory chronic GVHD can lead to astonishing clinical remissions (Landwehr-Kenzel et al. 2022).

However, manufacturing concerns due to the low abundancy of antigen-specific Treg cells in the peripheral blood have slowed the adoption of antigen-specific Treg cells in clinical practice (Landwehr-Kenzel et al., 2014). Until now, Treg therapy has been mainly performed using polyclonal Treg cells. Here, Treg cells are isolated together with other mononuclear cells from peripheral blood of donors and enriched *via* conventional Treg markers. Subsequently, the Treg cells are expanded by polyclonal stimulation *via* their T cell receptor/CD3 complex and additional co-stimulation using beads with anti-CD3 and anti-CD28 antibodies. Further, a high dose of IL-2 as a growth factor and the mTOR inhibitor rapamycin are added to the medium. However, there is only a small percentage of Treg cells present in the polyclonal product that exhibit the desired antigen-specificity and are allo-reactive and can, for example, recognize the kidney of a foreign organ donor and thus exert a protective influence on the transplant.

An alternative approach for the production of antigen-specific Treg cell products is genetic engineering of the cells. Via overexpression of transgenic T cell receptors (TCRs) or chimeric antigen receptors (CARs), polyclonal Treg cells can be made specific for a single target tissue. In particular, CARs represent a clinically relevant strategy. Chimeric antigen receptors (CAR) are tools to guide Treg cells towards specific tissue or cell types.

CARs are synthetic fusion genes, which typically combine the antigen-specific portion of a monoclonal antibody (called single-chain variable fragment [scFv]) with intracellular signalling domains that trigger T cell activation, usually via CD3 zeta. Poor performance of "1st generation" CARs have prompted the incorporation of additional co-stimulatory signaling domains, such as 4-1BB or CD28, to enhance T cell function. Although CAR-mediated immune synapses remain inefficient in comparison with T cell receptors (TCR), multiple 2nd generation CARs have yielded dramatic clinical responses in the treatment of B-cell malignancies. In Treg cells, CD28 has been favoured as a costimulatory domain in 2nd generation CARs (Dawson et al., 2020). An alternative strategy for the generation of antigen-specific T cell products from conventional T cells is the lentiviral transduction of T cells with so called T cell receptor fusion constructs (TruC) encoding for fusion proteins, in which antigen-binding domains are fused to a CD3 delta, gamma or epsilon subunit, which - upon expression - form an integral part of the CD3/TCR complex (Baeuerle et al., 2019).

However, manufacturing of CAR-redirected and TruC T cells is dominated by retroviral gene transfer. Production of retroviruses is costly and time consuming at clinical stages and requires high safety precautions. Non-viral approaches with transposase technology, such as PiggyBac or Sleeping Beauty, represent more affordable alternatives for gene transfer. However, they have not been adopted to Treg cells, presumably due to toxicity of plasmid transfection. Furthermore, two recent cases of CAR-derived lymphoma in a trial using hyperactive PiggyBac modified T cells (Micklethwaite et al., 2021; Bishop et al., 2021) attest a potential risk of mutagenesis when using transposase technology which may be attributed to random insertion of multiple transgene copies, genetic dysregulation by exogenous promoters or genetic scarring by transgene hopping of transposable elements (Wilson et al., 2021).

CRISPR-Cas gene editing represents an efficient alternative to enable site-specific gene transfer in Treg cells (Roth et al., 2018), which reduces the risk for insertional mutagenesis. Virus-free knock-in through electroporation of synthetic dsDNA and CRISPR-Cas9 RNPs was shown to allow efficient reprogramming of T cells such as Treg cells, including transfer of therapeutic TCRs and CARs (Roth et al., 2018; Schober et al., 2019; Muller et al., 2021; Kath et al., 2022). Studies using this approach usually integrate the transgenic antigen receptor into the TCR alpha constant gene (TRAC), because this leads to TCR replacement with low risk of alloreactivity and physiological regulation of the transgene, which has been associated with improved CAR T cell fitness in leukaemia models (MacLeod et al., 2017; Eyquem et al., 2017). Alternatively, it was show that the endogenous CD3 zeta chain (CD247) can also be used to express CARs in T cells and also Treg (WO2022/136551A1).

However, both approaches TRAC-KI and CD247-KI lead to CAR-Treg cells no longer carrying a TCR on the surface, as the TCR is replaced by the CAR. This makes cell expansion by repetitive anti-CD3/28 bead stimulation difficult, if not impossible. Thus, the absence of endogenous TCR-CD3 receptors causes a lack of stimulation capacity of the cells and a low, non-reproducible yield of antigen-specific Treg cells, hindering clinical and large-scale applications.

In view of the medical need and significant therapeutic potential of adoptive T cell therapy, in particular Treg cell therapy, for the prevention and/or treatment of unwanted and pathogenic immune reactions, straightforward and reproducible means for manufacturing antigen-specific T cells in sufficient cell numbers are urgently needed to enable improved clinical application of such cells. Further, efficient, affordable and in particular safe approaches for obtaining antigen specific T cells that do not bear the risks known in the prior art such as mutagenesis are required.

### SUMMARY OF THE INVENTION

In light of the prior art, the technical problem underlying the present invention is to provide improved or alternative means for immunotherapy of unwanted (pathogenic) immune reactions and cancerous diseases. A further object of the present invention is to provide a novel strategy for immunotherapy using genetically engineered therapeutic T cells.

In particular, one problem underlying the present invention is the provision of means for generating modified therapeutic T cells with an antigen-specific receptor, which are expandable ex vivo and overcome the disadvantages of the prior art. More specifically, one problem underlying the invention is the provision of means to generate antigen-specific modified Tregs that enable reliable expansion ex vivo. Another problem to be solved is the provision of means to enhance production capacity for genetically modified antigen-specific Tregs. Another problem underlying the invention is the provision of gene editing strategies for reprogramming of T cells to specific antigens, by genetically modifying the CD3/TCR complex.

These problems are solved by the features of the independent claims. Preferred embodiments of the present invention are provided by the dependent claims.

In one aspect, the invention relates to a nucleic acid construct comprising:
- a first nucleic acid sequence region, encoding an antigen binding domain, and
- a second nucleic acid sequence region, comprising a targeting sequence configured for integrating the construct in-frame with an endogenous CD3 epsilon gene of a human cell.

Surprisingly, the nucleic acid construct of the present invention enables easy and accurate in-frame integration of a transgene in the form of a sequence encoding an antigen binding domain into an endogenous CD3 epsilon gene of a T cell. Thereby, the in-frame integration of the nucleic acid construct provides a novel non-viral gene editing strategy for reprogramming T cells to specific antigens, while (i) preserving the functionality of the CD3/TCR receptor complex and (ii) maintaining T cell fitness. Further, the in-frame integration of the nucleic acid construct allows minimization of the transgene, which is beneficial for non-viral gene editing methods usually showing limited genetic cargo capacity. Thereby, the non-viral in-frame integration avoids the use of viral vectors and associated safety concerns, complexity and costly handling.

In contrast, conventional methods for obtaining antigen-specific T cells typically include the introduction of chimeric antigen receptors (CARs) to replace the CD3/TCR complex of the cells. However, as the CD3/TCR complex is essential for effective expansion of T cells ex vivo using conventional methods such as repetitive stimulation with antibody coated beads, the loss of CD3/TCR complex function hinders effective expansion of these cells and thus results in high costs and low efficiency of T cell immune therapy. In light of the typically low abundancy of antigen-specific T cells usually derived from the peripheral blood of a patient, the in-frame integration using the nucleic acid construct of the present invention allows both the straightforward non-viral generation of antigen-specific cells and the effective ex vivo expansion of these modified cells by repetitive stimulation. This allows the production of a high number of antigen-specific cells and thus increased efficiency of immunotherapy with such cells, while further reducing the costs, making these cells broadly applicable in various clinical settings.

Additionally, the nucleic acid construct of the present invention can be easily modified by a person skilled in the art in particular with regard to the sequence region encoding for the antigen binding domain in order to obtain T cells specific for a certain antigen, making the construct and resulting modified T cells specific for a particular antigen and thus applicable for a broad variety of indications, such as an unwanted (pathogenic) immune reactions, autoimmune disease or cancerous disease.

In one embodiment, the targeting sequence is configured for integrating the construct in-frame with an exon of the endogenous CD3 epsilon gene.

In some embodiments, the exon of the endogenous CD3 epsilon gene for insertion of said nucleic acid construct can be exon 1, exon 2, exon 3, exon 4, exon 5, exon 6, exon 7 or exon 8, preferably exon 1, 2, 3, 4, 5, or 6, more preferably exon 3 or exon 6, more preferably exon 3.

In one embodiment, the targeting sequence is configured for integrating the construct in-frame into exon 3 or exon 6 of the endogenous CD3 epsilon gene.

Surprisingly, the nucleic acid construct of the present invention allows integration of an antigen binding site directly in-frame into an exon of the endogenous CD3 epsilon gene, without resulting in a loss of function of the endogenous CD3 epsilon gene upon expression. This is advantageous for obtaining an antigen-specific and functional T cell fusion-protein - also termed receptor fusion construct (TruC) - comprising the functional CD3/TCR complex and an antigen binding domain. Further, in-frame integration into an exon of the endogenous CD3 epsilon gene results in regulation of the expression of the TRuC construct by an endogenous promoter, ensuring the maintenance of the functionality and fitness of the generated genetically modified T cell. Surprisingly, in particular, exon 3 and exon 6 resulted in effective expression of the TRuC in T cells.

In embodiments of the invention the target site of the CD3 epsilon gene, into which the first sequence region is integrated, is positioned downstream of a sequence encoding a CD3 epsilon signal peptide and upstream of an endogenous CD3 epsilon sequence (generating an N-terminal fusion after removal of the signal peptide).

The generation of an N-terminal fusion advantageously generates a fusion protein comprising an antigen binding domain and an endogenous CD3 epsilon protein, that is functional with respect to both the CD3 epsilon protein and the antigen-binding domain This N-terminal fusion protein can advantageously form an integral part of the endogenous CD3/TCR complex of a T cell, thereby generating an antigen specific T cell that can be efficiently expanded ex vivo.

In some cases, a sequence region upstream of the sequence of the integrated sequence region encoding for the antigen binding domain is transcribed together with the integrated sequence region. In some embodiments, means are therefore provided to separate any sequence upstream of the antigen binding domain during or after translation.

In some embodiments, a nucleic acid sequence region encoding a protein separation site, for example a self-cleavage peptide, a protease cleavage site or an internal ribosomal entry site (IRES), preferably a self-cleavage peptide, is positioned upstream of the integrated sequence region encoding for the antigen binding domain. The protein separation site is employed to separate any sequences upstream of the sequence region encoding for the antigen binding domain, that are translated together with said sequence, that may be dysfunctional or unwanted in the in-frame fusion peptide.

In one embodiment, the antigen binding domain encoding region is therefore separated from endogenous gene and/or mRNA sequence segments by a sequence encoding a self-cleavage peptide, proteolytic cleavage site, and/or an IRES site.

In one embodiment, a polypeptide cleavage site is selected from the group consisting of P2A, T2A, E2A and F2A.

In some embodiments, the self-cleavage peptide is preferably a 2A peptide, more preferably selected from the group consisting of a foot-and-mouth disease virus (FMDV) 2A peptide, an equine rhinitis A virus (ERAV) 2A peptide, a Thosea asigna virus (TaV) 2A peptide, a porcine tescho virus- 1 (PTV-I) 2A peptide, a Theilovirus 2A peptide, and an encephalomyocarditis virus 2A peptide.

In embodiments of the invention the targeting sequence of the second sequence region is configured for integration into a host genome by a gene editing technique, preferably CRISPR-Cas, zinc finger nucleases (ZFNs), integrases, site specific recombinases, meganucleases, homing endonucleases, or TALENs, more preferably a CRISPR-Cas12a derived from *Acidaminococcus species BV3L6* or CRISPR-Cas9 derived from *Streptococcus pyogenes.*

In embodiments of the invention the targeting sequence comprises an identical sequence to a recognition site of the endogenous DNA sequence into which integration is intended, preferably selected from the group consisting of a homology arm, a guide RNA target site, a restriction enzyme recognition site, a ZFN recognition site, a ZFN cleaving site, a TALEN DNA-binding site, a recombinase recognition site, an integrase site and/or a homing nuclease recognition site.

In certain embodiments, the targeting sequence, also termed "homology arm", comprises at least one sequence complementary to an endogenous genomic sequence of the target cell. In one embodiment, the targeting sequence of the second sequence region configured for integration into a host genome comprises a homology arm complementary to an endogenous integration site, described above, preferably complementary to a site within the endogenous CD3 epsilon gene.

In some embodiments, the nucleic acid construct comprises in 5' to 3' order a left homology arm, the nucleic acid sequence encoding the antigen binding domain and a right homology arm. In certain embodiments, the nucleic acid construct may additionally comprise one or more linkers in between the nucleic acid sequence encoding the antigen binding domain and the left and/or right homology arm and/or within the nucleic acid sequence encoding the antigen binding domain. In some embodiments the nucleic acid construct may optionally comprise a nucleic acid sequence encoding a self-cleaving peptide.

In one embodiment, the nucleic acid construct encodes an antigen binding domain, comprising:
- a left homology arm, preferably according to SEQ ID NOs 11 or SEQ ID NO 19 or a sequence with at least 80% sequence identity to SEQ ID NOs 11 or SEQ ID NO 19;
- a sequence region encoding an antigen-binding domain of a CAR that specifically binds to an antigen, preferably HLA, more preferably HLA-2 according to SEQ ID NOs 13 and 15 linked with a linker according to SEQ ID NO 14, or a sequence with at least 80% sequence identity to SEQ ID NOs 13, 14 and 15; and
- a right homology arm, preferably according to SEQ ID NOs 17 or SEQ ID NO 28 or a sequence with at least 80% sequence identity to SEQ ID NO 17 or SEQ ID NO 28.

In some embodiments, the nucleic acid construct encodes an antigen binding domain further comprising
- a sequence region encoding a self-cleaving, preferably according to SEQ ID NO 20, or a sequence with at least 80% sequence identity to SEQ ID NO 20

In one embodiment, a gene editing technique is used to create a double strand break, preferably at a target site in the CD3 epsilon gene, and the nucleic acid construct is incorporated into the target site by homology directed repair at the site of the double-strand break. A non-viral vector is preferably used for transferring the nucleic acid construct encoding the antigen binding domain fragment into the cell. In one embodiment, a delivery method is non-viral, preferably electroporation.

In one embodiment, the invention provides means to insert the transgene into a T cell using homology-directed repair (HDR). In one embodiment, the nucleic acid construct together with a CRISPR-Cas ribonucleoprotein (Cas) complexed with a guide RNA (gRNA) is transferred into the T cell. In one preferred embodiment the CRISPR-Cas is CRISPR-Cas9 or CRISPR-Cas12a, preferably CRISPR-Cas 12a.

Compared to retroviral gene transfer, the non-viral method according to the present invention represents a substantial improvement in comparison to the prior art, particularly with regard to the GMP-compliant production of the necessary starting materials. The production and purification of viral vectors according to GMP standards is complex and very cost intensive. In contrast, the acquisition of GMP-certified gene editing tools such as gRNAs and recombinant CRISPR-Cas proteins is simple and inexpensive.

Further genome editing through HDR by using CRISPR-Cas technology is advantageous for the specific and efficient and viral vector free insertion of large transgenes into target cells. It is advantageous that the viability and cell function of the transfected cells is maintained and allows a high yield of genetically modified cells for clinical application. As the gene transfer is sequence specific the method further provides targeted and safe integration of the nucleic acid construct, resulting in reduced mutagenic risk. In comparison, viral gene transfer or transposon technology randomly integrates DNA molecules into the genome. The random insertion of DNA fragments generates mutagenic risks, such as the development of malignant transformations.

In one embodiment, T cells are preferably co-electroporated with the gRNA targeting the CD3 epsilon gene. gRNA sequence is critical for a targeted genetic disruption. The gRNA disclosed herein (refer to Table 1) provides beneficial effects to the targeted genetic disruption by guiding a specific cleavage. Targeted disruption of CD3 epsilon gene leads to correct integration of the antigen binding domain by gene fusion, and results in sufficient translation of the functional in-frame fusion protein comprising the antigen binding domain and the endogenous CD3 epsilon protein. Such functional in-frame fusion protein is advantageous as it is efficiently transported to and anchored into the plasma membrane with the antigen binding domain being functionally presented at cell surface while maintaining CD3/TCR complex function. Such specific guiding for exact integration is critical for a precise synthesis of the in-frame fusion protein and its functionality, such as surface presentation and antigen-specificity, and is an advantageous feature of the guide RNA disclose herein over guide RNAs in prior art.

The present invention therefore provides gRNA sequences for targeting the CD3 epsilon gene that can be applied for T cells, and for obtaining antigen-specific T cells with maintained CD3/TCR complex function, which are expandable ex vivo and can be used for immunotherapy.

In embodiments of the invention the antigen binding domain is in the form of an antibody or antigen-binding fragment thereof.

In a preferred embodiment the antigen binding domain is an antigen-binding fragment, preferably a single chain fragment (scFv), a single variable fragment (ssFv), a single domain antibody (such as a VHH fragment), a Fab fragment, a F(ab')2 fragment, a fragment produced by a Fab expression library, an anti-idiotypic antibody or an epitope-binding fragment or a combination thereof, preferably a single chain fragment (scFv).

The use of an antigen-binding fragment domain such as an scFV fragment proves to be particularly advantageous because these fragments are small, can be easily modified for different antigens and further can be encoded by a small nucleic acid fragment, and are thus easy to integrate into the endogenous CD3 epsilon gene of a T cell by using non-viral methods.

In one embodiment, the invention relates to an in-frame fusion protein comprising an antigen binding domain and an endogenous CD3 epsilon protein as described herein, wherein the antigen-binding domain comprises a variable heavy chain (VH), said VH comprising:
- a heavy chain complementary determining region 1 (H-CDR1) with at least 80% sequence identity to SEQ ID NO 52 (GVTLSDY),
- a heavy chain complementary determining region 2 (H-CDR2) with at least 80% sequence identity to SEQ ID NO 52 (RNDGSD), and
- a heavy chain complementary determining region 3 (H-CDR3) with at least 80% sequence identity to SEQ ID NO 53 (NGESGPLDYWYFDL),
and a variable light chain (VL), said VL comprising:
- a light chain complementary determining region 1 (L-CDR1) with at least 80% sequence identity to SEQ ID NO 54 (QASQDISNYLN),
- a light chain complementary determining region 2 (L-CDR2) with at least 66% sequence identity to SEQ ID NO 55 (DASNLET), and
- a light chain complementary determining region 3 (L-CDR3) with at least 80% sequence identity to SEQ ID NO 56 (QQYSSFPLT).

In one embodiment, the invention relates an in-frame fusion protein comprising an antigen binding domain and an endogenous CD3 epsilon protein as described herein, comprising a VH domain that comprises CDR sequences of SEQ ID NO. 51, SEQ ID No. 52 and SEQ ID NO. 53, and a VL domain that comprises CDR sequences of SEQ ID NO. 54, SEQ ID NO 55, and SEQ ID NO. 56.

In some embodiments, the sequence variants with 80% or more sequence identity to the specific antigen binding sequence of SEQ ID 47 maintain HLA-A2 binding with essentially the same or similar functional properties as VH and VL domains with the specific sequences of SEQ ID NO 48 and 50, i.e., the HA-A2 binding is essentially the same or similar with respect to affinity, specificity and/or epitope binding mode.

In one embodiment of the invention the antigen binding domain is specific for an autoantigen, an alloantigen or a cancer antigen.

In one embodiment the antigen-binding domain is specific for an autoantigen or alloantigen.

In embodiments, the autoantigen or alloantigen is an HLA protein, preferably selected from a group of HLA proteins including HLA-A, HLA-B, HLA-DR as well as HLA associated antigens such as Mic A, or Mic B. In a preferred embodiment the autoantigen or alloantigen is selected from HLA-A2, HLA-B7, HLA-DR as well as HLA associated antigens, such as Mic A, or Mic B.

In one embodiment, the antigen binding domain is specific for an HLA protein, preferably HLA-A2.

In one embodiment the antigen binding domain is specific to a cancer antigen, wherein the cancer antigen is selected from the group of ErbB proteins such as EGFR and HER2/neu and B-lymphocyte antigens such as CD19. In a preferred embodiment cancer antigen is selected from HER2/neu or CD19.

In a further aspect the invention relates to a genetically modified human T cell, comprising an exogenous nucleic acid sequence region encoding an antigen binding domain integrated in-frame into an endogenous CD3 epsilon gene.

In embodiments of the invention the genetically modified human T cell is a regulatory human T cell (Treg).

In embodiments the invention relates to the genetically modified human T cell, wherein a CD3 epsilon protein is expressed as an in-frame fusion protein comprising the antigen binding domain and endogenous CD3 epsilon protein.

In embodiments the invention relates to the genetically modified human regulatory T cell (Treg), wherein a CD3 epsilon protein is expressed as an in-frame fusion protein comprising the antigen binding domain and endogenous CD3 epsilon protein.

In embodiments the invention relates to the genetically modified human T cell, wherein T cell receptor (TCR) complex function is maintained in the presence of the in-frame fusion protein comprising the antigen binding domain and endogenous CD3 epsilon protein.

In embodiments the invention relates to the genetically modified human regulatory T cell (Treg), wherein T cell receptor (TCR) complex function is maintained in the presence of the in-frame fusion protein comprising the antigen binding domain and endogenous CD3 epsilon protein.

Advantageously, the genetically modified T cells of the present invention show maintained functionality of the CD3/TCR receptor complex, cell fitness and antigen-specificity. The preservation of the CD3/TCR complex function receptors enables the maintenance of the stimulability of the genetically modified antigen-specific T cells and thus the efficient expansion ex vivo to a similar extent as unmodified T cells with a functional CD3/TCR complex. In contrast within conventionally modified T cells such as CAR-T cells the CD3/TCR complex is replaced by the CAR resulting in a loss of function and stimulability limiting effective expansion and thus clinical applicability of these cells.

Further, the genetically modified T cells of the present invention can be easily modified with regards to the antigen-specificity of the in-frame fusion protein expressed. Thereby, T cells specific for a certain antigen can be obtained for use in the prevention and or treatment of an unwanted (pathogenic) immune reaction, autoimmune disease or cancerous disease associated with the specific antigen.

In addition, it is advantageous that the genetically modified T cells show comparable functionality to unmodified T cells with regards to signal transduction, expression of activation markers and suppression of the proliferation of conventional T cells assays.

In embodiments the invention relates to the genetically modified human T cell, wherein the fusion protein is expressed under the control of an endogenous CD3 epsilon promoter.

In embodiments the invention relates to the genetically modified human regulatory T cell (Treg), wherein the fusion protein is expressed under the control of an endogenous CD3 epsilon promoter.

The endogenous promoter is intrinsically regulated and/or induced, e.g., by cellular signals, from the T cell. It is a particular advantage of the present invention that it employs endogenous promoter expression. Regulation of the expression of the integrated nucleic acid construct by an endogenous promoter ensures the maintenance of the functionality and fitness of the generated genetically modified T cell. Further, by omitting exogenous promoters, mutagenic risk can be reduced caused by gene dysregulation via regulatory elements. In addition, due to the absence of an exogenous promoter, the nucleic acid construct of the invention is smaller and results in a particularly effective transfer and integration of the nucleic acid sequences by non-viral methods.

In embodiments the invention relates to the genetically modified human T cell, wherein the exogenous nucleic acid sequence region encoding a recombinant antigen binding domain is integrated in-frame into an exon of the endogenous CD3 epsilon gene.

In embodiments the invention relates to the genetically modified human regulatory T cell (Treg), wherein the exogenous nucleic acid sequence region encoding a recombinant antigen binding domain is integrated in-frame into an exon of the endogenous CD3 epsilon gene.

In a further embodiment the exogenous nucleic acid sequence region encoding a recombinant antigen binding domain is integrated in-frame into exon 3 or exon 6 of the endogenous CD3 epsilon gene.

In embodiments the invention relates to the genetically modified T cell, wherein the target site of the CD3 epsilon gene, into which the first sequence region is integrated, is positioned downstream of a sequence encoding a CD3 epsilon signal peptide and upstream of an endogenous CD3 epsilon sequence (generating an N-terminal fusion after removal of the signal peptide).

In embodiments the invention relates to the genetically modified regulatory T cell (Treg), wherein the target site of the CD3 epsilon gene, into which the first sequence region is integrated, is positioned downstream of a sequence encoding a CD3 epsilon signal peptide and upstream of an endogenous CD3 epsilon sequence (generating an N-terminal fusion after removal of the signal peptide).

In embodiments the invention relates to the genetically modified human T cell, wherein the recombinant antigen binding domain binds to an autoantigen, an alloantigen of a transplant or a cancer antigen.

In embodiments the invention relates to the genetically modified human regulatory T cell (Treg), wherein the recombinant antigen binding domain binds to an autoantigen, an alloantigen of a transplant or a cancer antigen.

In one embodiment the antigen-binding domain binds to an autoantigen or alloantigen, wherein the autoantigen or alloantigen is selected from a group of HLA proteins including HLA-A, HLA-B, HLA -C, HLA-E, HLA-DR, HLA-DQ, as well as HLA associated antigens such as Mic A, or Mic B. In a preferred embodiment the autoantigen or alloantigen is selected from HLA-A2, HLA-B7, HLA-B27, HLA-DR as well as HLA associated antigens, such as Mic A, or Mic B.

In one embodiment the antigen binding domain binds to an HLA protein, preferably HLA-A2.

In one embodiment the antigen binding domain binds to a cancer antigen, wherein the cancer antigen is selected from the group of ErbB proteins such as EGFR and HER2/neu and B-lymphocyte antigens such as CD19. In a preferred embodiment cancer antigen is selected from HER2/neu or CD19.

In a further aspect, the invention relates to a genetically modified T cell for use as a medicament for the prevention and/or treatment of an unwanted (pathogenic) immune reaction, preferably an unwanted (pathogenic) immune reaction prior to and/or after an allogeneic transplantation, more preferably for the prevention and/or treatment of graft-versus-host disease (GvHD).

In a further aspect, the invention relates to a genetically modified regulatory T cell (Treg) as described herein for use as a medicament for the prevention and/or treatment of an unwanted (pathogenic) immune reaction, preferably an unwanted (pathogenic) immune reaction prior to and/or after an allogeneic transplantation, more preferably for the prevention and/or treatment of graft-versus-host disease (GvHD).

In a further aspect, the invention relates to a genetically modified T cell as described herein for use as a medicament for the prevention and/or treatment of a cancerous disease.

In a further aspect, the invention relates to a genetically modified regulatory T cell (Treg) as described herein for use as a medicament for the prevention and/or treatment of an autoimmune disease.

In a further aspect, the invention relates to a polypeptide expressed from a genetically modified cell as described herein as a fusion protein comprising a recombinant antigen binding domain and endogenous CD3 epsilon protein.

In a further aspect, the invention relates to a pharmaceutical composition comprising a genetically modified cell as described herein and a pharmaceutically acceptable carrier.

In a further aspect, the invention relates to a method for preparing a therapeutic genetically modified T cell as described herein, comprising repetitive stimulation of a genetically modified regulatory T cell via the TCR/CD3 complex and CD28 co-receptor, wherein said therapeutic genetically modified regulatory T cell exhibits in essence unmodified expansion dynamics in culture (+/- 50%) in comparison to an unmodified T cell.

In a further aspect, the invention relates to a method for preparing a therapeutic genetically modified regulatory T cell (Treg) as described herein , comprising repetitive stimulation of a genetically modified regulatory T cell via the TCR/CD3 complex and CD28 co-receptor, wherein said therapeutic genetically modified regulatory T cell exhibits in essence unmodified expansion dynamics in culture (+/- 50%, +/- 40%,+/- 30%,+/- 20%,+/- 10%, or +/- 5%) in comparison to an unmodified regulatory T cell.

The term unmodified expansion dynamics in culture refers to the beneficial properties of the modified cells during cultivation and repetitive stimulation. The modified cells of the invention are not any worse in expansion dynamics in culture, compared to cells without the modification (and thus the inventive cells exhibit an improvement over alternative cells lacking a TCR; refer to comparative example 1, below). In embodiments, expansion dynamics refers to growth rate of the cells and/or maintenance of Treg properties during cultivation. The examples provide a more detailed representation of the necessary properties below.

In light of the in general low abundancy of expandable unmodified antigen-specific T cells within the peripheral blood and lack of expandability of conventionally modified antigen-specific T cells such as CAR-T cells, it is particularly advantageous that the genetically modified T cells can be efficiently expanded by repetitive stimulation via the TCR/CD3 complex and CD28 co-receptor. The repetitive stimulation thereby provides efficient expansion to high cell numbers and high reproducibility while maintaining fitness of the genetically modified T cells. Sufficient and reproducible expansion of antigen-specific T cells is an important factor for enabling clinical applicability of the cells and further cost reduction of such applications.

All features described in the present specification may be employed to define any other embodiment or aspect of the invention, for example, features used to describe the genetically modified T cell may be used to describe the nucleic acid construct, the polypeptide, the pharmaceutical composition or the method for preparing a therapeutic genetically modified T cell, and vice versa. Similarly, features used to describe the methods of the invention may be used to describe the cells, constructs, polypeptides or compositions, and vice versa.

### DETAILED DESCRIPTION OF THE INVENTION

The present invention provides a strategy for immune cell therapy that uses gene editing methods to preferably integrate one or more antigen binding domains under the control of an endogenous CD3 epsilon promoter enabling cell-specific and endogenously controlled expression in therapeutic T cells. The various aspects and embodiments of the invention, in addition to their advantages over the prior art, are described above.

A "T cell" also termed "T lymphocyte" is an immune cell belonging to the group of lymphocytes. A T cell can be a thymocyte, immature T lymphocyte, mature T lymphocyte, resting T lymphocyte, cytokine-induced killer cell (CIK cell) or activated T lymphocyte. T cells originate from the bone marrow and migrate via the blood stream to the thymus, where they generate T cell receptors (TCR) and undergo a positive and negative selection in which the cells that show high affinity to endogenous proteins are degraded. T cells may be a T helper (Th; CD4+ T cell) cell, for example a T helper (Th) cell, such as a TH1, TH2, TH3, TH17, TH9 or TFH cell. The T cell can be a cytotoxic T cell (CTL; CD8+ T cell), CD4+CD8+ T cell, CD4 CD8 T cell, or a regulatory T cell (reg) or any other subset of T cells such as a cytokine-induced killer (CIK) cell which is typically a CD3- and CD56-positive, non-major histocompatibility complex (MHC)- restricted, natural killer (NK)-like T lymphocyte. The T cell can be a naive, effector, memory, effector storage, central storage, memory stem T cell. The T cell can be an umbilical cord blood cell. The T cell can be a peripheral lymphocyte. A T-cell can be derived and expanded from peripheral mononuclear blood cells (PBMNCs). The T-cell may be autologous with respect to an individual to whom it is to be administered. The T-cell may be allogeneic with respect to an individual to whom it is to be administered.

A regulatory T cell (Treg) is an immunosuppressive usually CD4+ T cell that prevents autoimmune diseases by maintaining tolerance to self-antigens also termed "autoantigens", modulates and ends overshooting immune responses, ensures tissue regeneration and regulates bacterial homeostasis at mucosal surfaces. The activation of Treg cells is antigen specific. Upon activation Treg cells secrete various cytokines including without limitation interleukin 10 (IL-10), transforming growth factor β (TGF-β) and interleukin 35 (IL-35). This results in e.g., increase of the activation level of naïve T-cells, inhibition of the proinflammatory function of antigen-presenting cells (APCs) and downregulation of the induction and proliferation of T effector cells. In circulation, 2-5% of the peripheral blood's CD4+ T cells represent Treg cells characterized by canonical markers, such as high expression the transcription factor Forkhead-Box-Protein P3 (Foxp3) and the high-affinity interleukin 2 (IL-2) receptor chain, CD25, as well as low expression of the interleukin 7 (IL-7) receptor alpha chain, CD127.

A "T cell receptor" (TCR) is a protein complex found on the surface of T cells, that is responsible for recognizing and binding fragments of antigens bound to major histocompatibility complex (MHC) molecules e.g., on the surface of APCs. The TCR is a heterodimer, which comprises a TCR alpha and TCR beta protein chain or a TCR gamma and a TCR delta protein chain. T cells with an TCR composed of an TCR alpha and an TCR beta protein chain account for approx. 95% of the T cell population in the peripheral blood. The subunits of the heterodimer each consist of a constant domain (C) and a variable domain (V), a transmembrane domain and a short C-terminal cytoplasmic region. The N-terminal ends of the chains belonging to the C domain penetrate the cell membrane into the cytoplasmic space and anchor the receptor. The two subunits are extracellularly linked by a disulfide bridge in the constant region. The "TCR complex" also termed "TCR/CD3 complex" is formed by the TCR and CD3 comprising CD3 gamma, CD3 delta, CD3 epsilon and CD3 zeta subunits. By binding an antigen presented by MHC II (antigen/MHC II complex) on the surface of APC or by MHC I on any other cell type (antigen/MHC I complex) the TCR-mediated signals are transmitted by the CD3 chains zeta, delta, epsilon, and gamma across the cell membrane activating the T cell. All CD3 chains contain immune receptor tyrosine-based activation motifs (ITAMs) in their cytoplasmic domain. The genes encoding the epsilon, gamma and delta polypeptides are located in the same cluster on chromosome 11.

As used herein, the terms "polynucleotide", "nucleic acid", "nucleic acid molecule" or "nucleic acid construct" refers to a polymeric form of nucleotides (nt) of any length, wherein the nucleotides can be either deoxyribonucleotides (DNA) or ribonucleotides (RNA), or analogs thereof. The terms include, without limitation, DNA, messenger RNA (mRNA), RNA, genomic RNA (gRNA), transfer RNA (tRNA), ribosomal RNA (rRNA), microRNA (miRNA), small interfering RNA (siRNA), single guide RNA (sgRNA), piwi-interacting RNA (piRNA), small nuclear RNA (snRNA),plus strand RNA (RNA(+)), minus strand RNA (RNA(-)), genomic DNA (gDNA), complementary DNA (cDNA), recombinant polynucleotides, branched polynucleotides, plasmids, nucleic acid probes and primers. Polynucleotides include single and double stranded polynucleotides. Preferably, polynucleotides of the invention include polynucleotides or variants having at least about 50%, 55%, 60%, 65%, 70%, 75%, 80%, 85%, 90%, 95%, 96%, 97%, 98%, 99% or 100%) sequence identity to any of the reference sequences described herein, typically where the variant maintains at least one biological activity of the reference sequence.

As used herein, "sequence identity", "identity", "sequence homology" or "homology" in the context of two nucleic acid sequences makes reference to a specified percentage of residues in the two sequences that are the same when aligned for maximum correspondence over a specified comparison window, as measured by sequence comparison algorithms or by visual inspection.

As used herein, "percent (%) sequence identity", "sequences with % identity", "percent (%) sequence homology" or "sequences with % homology" to a specific "reference sequence" (e.g., to SEQ ID No 1) means the percentage of nucleotides or amino acids in a certain sequence that are identical with the nucleotides or amino acids of the reference sequence and is determined by comparing the two optimally aligned sequences over a comparison window wherein the portion of the polynucleotide or amino acid sequences in the comparison window may include additions or deletions (i.e., gaps) as compared to the reference sequence (which does not include additions or deletions) for optimal alignment of the two or more sequences. The percentage is calculated by determining the number of positions at which the identical nucleic acid base or amino acid residue occurs in the two or more sequences to yield the number of matched positions, dividing the number of matched positions by the total number of positions in the window of comparison, and multiplying the result by 100 to yield the percentage of sequence identity. Included are nucleotides and polypeptides having at least about 50%, 55%, 60%, 65%, 70%, 75%, 80%, 85%, 90%, 95%, 96%, 97%, 98%, 99% or 100% sequence identity to any of the reference sequences described herein, typically where the polypeptide variant maintains at least one biological activity of the reference polypeptide.

Any suitable methods of alignment of sequences for determination the percent sequence identity may be employed and are known to a person skilled in the art. The determination of percent identity between any two or more sequences can be accomplished using a publicly available mathematical algorithm. Computer software implementations of these mathematical algorithms include but are not limited to: ClustaIW algorithm (VNTI software, InforMax Inc.), ALIGN (Version 2.0), GAP (Genetics Computer Group, software; now available via Accelrys on http://www.accelrys.com), BESTFIT, BLAST^{®}, FASTA, and TFASTA in the Wisconsin Genetics Software Package, Version 8 (available from Genetics Computer Group (GCG), Madison, Wis., USA) and Multiple sequence alignments MUSCLE (EMBL's European Bioinformatics Institute (EMBL-EBI), Cambridgeshire, UK). Alignments using these programs can be performed using the default parameters. Software for performing BLAST^{®} analyses is publicly available through the National Center for Biotechnology Information. A sequence database can be searched using the nucleic acid sequence of interest. Algorithms for database searching are typically based on the BLAST software (Altschul *et al.*, 1990). In some embodiments, the percent homology or identity can be determined along the full-length of the nucleic acid.

It will be appreciated by those of ordinary skill in the art that, as a result of the degeneracy of the genetic code, there are many nucleotide sequences that encode an in-frame fusion protein as described herein. Some of these polynucleotides bear minimal homology or sequence identity to the nucleotide sequence of any native gene. Nonetheless, polynucleotides that vary due to differences in codon usage are specifically contemplated by the present invention. Deletions, substitutions and other changes in sequence that fall under the described sequence identity are also encompassed in the invention.

Protein sequence modifications, which may occur through substitutions, are also included within the scope of the invention. Substitutions as defined herein are modifications made to the amino acid sequence of the protein, whereby one or more amino acids are replaced with the same number of (different) amino acids, producing a protein which contains a different amino acid sequence than the primary protein. Substitutions may be carried out that preferably do not significantly alter the function of the protein. Like additions, substitutions may be natural or artificial. It is well known in the art that amino acid substitutions may be made without significantly altering the protein's function. This is particularly true when the modification relates to a "conservative" amino acid substitution, which is the substitution of one amino acid for another of similar properties. Such "conserved" amino acids can be natural or synthetic amino acids which because of size, charge, polarity and conformation can be substituted without significantly affecting the structure and function of the protein. Frequently, many amino acids may be substituted by conservative amino acids without deleteriously affecting the protein's function.

In general, the non-polar amino acids Gly, Ala, Val, lie and Leu; the non-polar aromatic amino acids Phe, Trp and Tyr; the neutral polar amino acids Ser, Thr, Cys, Gin, Asn and Met; the positively charged amino acids Lys, Arg and His; the negatively charged amino acids Asp and Glu, represent groups of conservative amino acids. This list is not exhaustive. For example, it is well known that Ala, Gly, Ser and sometimes Cys can substitute for each other even though they belong to different groups.

Substitution variants have at least one amino acid residue in the antibody molecule or antibody fragment removed and a different residue inserted in its place. The sites of greatest interest for substitutional mutagenesis include the hypervariable regions, but framework alterations are also contemplated. If such substitutions result in a change in biological activity, then more substantial changes, denominated "exemplary substitutions" in the table immediately below, or as further described below in reference to amino acid classes, may be introduced and the products screened.

### Potential Amino Acid Substitutions:

| **Original residue** | **Preferred conservative substitutions** | **Examples of exemplary substitutions** |
|---|---|---|
| Ala (A) | Val | Val; Leu; Ile |
| Asg (R) | Lys | Lys; Gin; Asn |
| Asn (N) | Gln | Gln; His; Asp, Lys; Arg |
| Asp (D) | Glu | Glu; Asn |
| Cys (C) | Ser | Ser; Ala |
| Gln (Q) | Asn | Asn, Glu |
| Glu (E) | Asp | Asp; Gln |
| Gly (G) | Ala | Ala |
| His (H) | Arg | Asn; Gln; Lys; Arg |
| Ile (I) | Leu | Leu; Val; Met; Ala; Phe; Norleucine |
| Leu (L) | Ile | Norleucine; Ile; Val; Met; Ala; Phe |
| Lys (K) | Arg | Arg; Gln; Asn |
| Met (M) | Leu | Leu; Phe; Ile |
| Phe (F) | Tyr | Leu; Val; Ile; Ala; Tyr |
| Pro (P) | Ala | Ala |
| Ser (S) | Thr | Thr |
| Thr (T) | Ser | Ser |
| Trp (W) | Tyr | Tyr; Phe |
| Tyr (Y) | Phe | Trp; Phe; Thr; Ser |
| Val (V) | Leu | Ile; Leu; Met; Phe; Ala; Norleucine |

Substantial modifications in the biological properties of the antibody or antibody fragment are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain.

Conservative amino acid substitutions are not limited to naturally occurring amino acids, but also include synthetic amino acids. Commonly used synthetic amino acids are omega amino acids of various chain lengths and cyclohexyl alanine which are neutral non-polar analogues; citrulline and methionine sulfoxide which are neutral non-polar analogues, phenylglycine which is an aromatic neutral analog; cysteic acid which is a negatively charged analog and ornithine which is a positively charged amino acid analogue. Like the naturally occurring amino acids, this list is not exhaustive, but merely exemplary of the substitutions that are well known in the art.

An "antigen (Ag)" refers to a compound, composition, or substance that can bind to an antibody. An antigen stimulating an "immune response" or "immune reaction" such as the production antibodies or a T cell response in an animal or human subject is also referred to as "immunogen". Antigens and/or immunogens usually comprise protein, lipid and/or carbohydrate structures. An antigen usually has several antigenic substructures, which are the region of an antigen to which a binding agent binds, and which are referred to as "determinants" or "epitopes". Thereby, epitopes can be formed both from contiguous structures such as an amino acid sequence or noncontiguous structures such as two or more amino acid sequences juxtaposed by tertiary folding of a protein. Therefore, epitopes can be composed on two distinct proteins in proximity or a protein and another immunogen as a complex.

The term "autoantigen" refers to endogenous substance or structure, that is usually not recognized by T cells due to their selection within the thymus resulting in the ability to discriminate between physiological, endogenous structures and pathological and/or foreign structures. If not recognized as healthy endogenous structure by T cells, autoantigens can lead to an "unwanted immune reaction" such as an "autoimmune reaction" by pathological T cell activation and antibody production which can manifest as an autoimmune disease. Autoantigens involved in the manifestation of autoimmune diseases include without limitation the IgA immunoglobulin in IgA-nephropathy, the Fc part of IgG and cyclic citrullinated peptide (CCP) in rheumatoid arthritis; thyrotropin (TSH) receptor and thyroid peroxidase (microsomal) in Graves' disease; acetylcholine receptor (AChR) and muscle-specific kinase (MUSK) in Myasthenia gravis; insulin, islet cell surface antigens, glutamate decarboxylase (predominantly GAD65, but also GAD67), zinc transporter 8 and tyrosine phosphatase IA-2 in type 1 diabetes; thrombin, ribonucleoproteins, double-stranded DNA, snRNP core proteins and histones in systemic lupus erythematosus; phospholipid in antiphospholipid syndrome; thyroglobulin and thyroid peroxidase (microsomal) in Hashimoto's thyroiditis; nucleoporin 62, Sp100 nuclear antigen, nucleoporin 210 kDa in primary biliary cirrhosis; voltage-gated calcium channel (P/Q-type) in Lambert-Eaton myasthenic syndrome; Type IV collagen in Goodpasture's syndrome; DNA topoisomerase in scleroderma; centromere proteins A, B and C in CREST syndrome; ribonucleoprotein in Sjögren's syndrome and reticulin, endomysium and tissue transglutaminase in Celiac disease. Autoimmune diseases include without limitation rheumatoid arthritis, Graves' disease, Myasthenia gravis, type 1 diabetes, systemic lupus erythematosus, antiphospholipid syndrome, Hashimoto's thyroiditis, primary biliary cirrhosis, Lambert-Eaton myasthenic syndrome, Goodpasture's syndrome, Sjögren's syndrome, Celiac disease, Multiple sclerosis, Ulcerative colitis, Pemphigus vulgaris, Polymyositis, Scleroderma, Systemic vasculitides, Alopecia areata, autoimmune liver disease (AILD), autoimmune hepatitis, primary sclerosing cholangitis, autoimmune hemolytic anemia, ord thyroiditis, bullous pemphigoid, dermatomyositis, eosinophilic granulomatosis with polyangiitis (EGPA), Guillain-Barré syndrome, inhibitory hemophilia, immune thrombocytopenia, Lambert-Eaton syndrome, Adamantiades-Behçet's disease, narcolepsy, primary chronic polyarthritis, polychondritis, psoriasis, Schönlein-Henoch purpura, rheumatic fever, Giant cell arteritis (RZA), SAPHO syndrome, Stiff-Man syndrome, Type A gastritis, vitiligo and Wegener's granulomatosis.

Autoantigens further play a role in Graft-versus-host reaction (GVHR) also termed "Graft-versus-host disease" (GVHD), which is an unwanted systemic, cytotoxic immune reaction of implanted or transfused immune cells against the host organism. Transplanted T cells recognize the recipient's autoantigens as foreign, triggering cellular immune responses in the recipient organism. As a result, specific cytotoxic T cells and antibodies directed against the host are produced. In addition, reactions of the innate immune system are also suspected. In immunocompromised hosts, further, severe diseases such as hepatosplenomegaly, atrophy of lymphoid organs, diarrhea, skin lesions, and cachexia may occur. In chronic graft-versus-host reaction, both alloreactive and autoreactive immunologic processes occur. Impaired peripheral immune tolerance is associated with a deficiency of Treg cells. As a consequence, a chronic inflammatory reaction with consecutive fibrosis develops. GVHD plays a particularly important role in allogeneic stem cell transplantation and rarely also occurs after transfusions in patients with severe congenital or acquired immunodeficiency.

An "alloantigen" is an antigen produced by an individual that elicits an immune response in an individual of the same biological species. Typical alloantigens include surface antigens of erythrocytes and lymphocytes and allogenic transplants. Alloantigens are possible triggers of an "unwanted immune reaction" resulting in blood group incompatibilities and transplant rejections. The most important group of alloantigens are human leukocyte antigens (HLA) also termed major histocompatibility complex (MHC) molecules. HLA are glycoproteins anchored in the cell membrane and are classified as immunoglobulins. HLA are classified into class I MCH molecules and class II MHC molecules. Class I includes HLA-A, HLA-B, HLA-C, HLA-E, HLA-F, HLA-G and subgroups thereof. Class II includes HLA-DP, HLA-DQ, HLA-DR and subgroups thereof. Class I MHC molecules are located as transmembrane glycoproteins on the surface of all nucleated cells. Class II MHC molecules are typically found on APCs, such as B cells, phagocytosing cells (e.g., macrophages), and dendritic cells. In general, both classes of HLA molecules serve to bind peptides and present them on the cell surface to the cells of the immune system. In particular HLA antigens HLA-A, HLA-B, HLA-C, HLA-DRB1, HLA-DQB1 and HLA-DPB1 play a major role in allogenic transplant rejection. Certain HLA antigens also play a role in autoimmune diseases including HLA-B27 in ankylosing spondylitis, reactive arthritis and acute anterior uveitis; HLA-B47 in 21-hydroxylase deficiency; HLA-DR2 in systemic lupus erythematosus; HLA-DR3 in autoimmune hepatitis, primary Sjögren syndrome, diabetes mellitus type 1 and systemic lupus erythematosus; HLA-DR4 in rheumatoid arthritis and diabetes mellitus type 1 and HLA-DQ2 and HLA-DQ8 in Coeliac disease. Alloantigens may be classified into major alloantigens, such as MHC mismatches, but minor mismatches may also occur, e.g., donor A has a mutation in protein X, a fragment of the mutated protein X is presented via a matched HLA, thereby inducing an immune response in T cells, because the mutated fragment can be recognized as non-self.

The term "immune reaction" refers to the reaction of the immune system of a subject to the confrontation with an antigen. Thereby, the immune system usually distinguishes between endogenous structures such as autoantigens and exogenous structures. An "unwanted immune reaction" refers to the reaction of the immune system of a subject against an autoantigen or alloantigen as stated above. The terms "pathogenic" and "pathogenic immune reaction" refer to reactions of the immune system that are harmful to the body. Such reactions include e.g., the autoimmune reactions and diseases listed under the term autoantigen as well as GVHD or transplant rejection due to reaction to alloantigens.

A "cancer antigen", "Cancer-associated antigen" or "tumor antigen" is expressed interchangeably on the surface of cancer cells, either completely or as a fragment (e.g., MHC / peptide). Tumor antigens are either specifically expressed on tumor cells and not found on non-pathogenic cells or abnormally expressed, e.g., at least twice above the level found in non-pathogenic cells. Antigens, specifically found on tumor cells, may appear foreign to the immune system and their presence may cause the immune cells to attack the transformed tumor cells. Tumor antigen refers to an antigen that is commonly found in a specific hyperproliferative disease. In one aspect, the antigen of hyperproliferative disease of the present invention is a primary or metastatic melanoma, thymoma, lymphoma, sarcoma, lung cancer, liver cancer, non-Hodgkin's lymphoma, Hodgkin's lymphoma, Leukemia, uterine cancer, cervical cancer, bladder cancer, derived from cancers such as kidney and breast cancer, prostate cancer, ovarian cancer, adenocarcinoma such as pancreatic cancer and the like. Cancer associated antigen include without limitation CD19, APRIL/TNFSF13, Mic A/B, T cell associated antigens: CD3, CD5, CD7, Folate receptor alpha (FRa), ERBB2 (HER2/neu), EphA2, IL-13Ra2, epidermal growth factor receptor (EGFR), Mesothelin, TSHR, CD123, CD22, CD30, CD171, CS-1, CLL-1, CD33, EGFRvlll, GD2, GD3, BCMA, Tn Ag, prostate specific membrane antigen (PSMA), ROR1, FLT3, FAP, TAG72, CD38, CD44v6, CEA, EPCAM, B7H3, KIT, interleukin-11 receptor a (IL-11Ra), PSCA, PRSS21, VEGFR2, LewisY, CD24, platelet-derived growth factor receptor-beta (PDGFR-beta), SSEA-4, CD20, MUC1, NCAM, Prostase, PAP, ELF2M, Ephrin B2, IGF-I receptor, CAIX, LMP2, gp100, bcr-abl, tyrosinase, Fucosyl GM1, sLe, GM3, TGS5, HMWMAA, o-acetyl-GD2, Folate receptor beta, TEM1/CD248, TEM7R, CLDN6, GPRC5D, CXORF61, CD97, CD179a, ALK, Polysialic acid, PLAC1, GloboH, NY-BR-1, UPK2, HAVCR1, ADRB3, PANX3, GPR20, LY6K, OR51E2, TARP, WT1, NY-ESO-1, LAGE-1a, MAGE-A1, legumain, HPV E6,E7, MAGE A1, ETV6-AML, sperm protein 17, XAGE1, Tie 2, MAD-CT-1, MAD-CT-2, Fos-related antigen 1, p53, p53 mutant, prostein, survivin, telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, mut hsp70-2, CD79a, CD79b, CD72, LAIR1, FCAR, LILRA2, CD300LF, CLEC12A, BST2, EMR2, LY75, GPC3, FCRL5, IGLL1, , and derivates thereof comprising post-translational modifications of the polypeptide, for example, glycosylations, ubiquitination, acetylations, phosphorylations and the like, as well as other modifications known in the art, both naturally occurring and non-naturally occurring.

According to the present invention any suitable autoantigen, alloantigen or cancer antigen can be selected depending on the type of medical condition, wherein the genetically modified T cell according to the present invention is intended to be used for. The genetically modified T cells according to the present invention are preferably for use in the prevention and or treatment of an unwanted (pathogenic) immune reaction, such as an unwanted immune reaction prior to and/or after an allogenic transplantation and/or a cancerous disease. In preferred embodiments the genetically modified T cells are preferably for use for the prevention and/or treatment of graft-versus-host disease (GVHD).

"Specific binding" is to be understood as selective binding of an antigen binding domain to a specific antigen, whereby the skilled person is clearly aware of various experimental procedures that can be used to test binding and binding specificity. Methods for determining equilibrium association or equilibrium dissociation constants are known in the art. Some cross-reaction or background binding may be inevitable in many antigen binding domain antigen interactions; this is not to detract from the "specificity" of the binding between the antigen binding region and antigen. By way of example, "specific binding" describes binding of an e.g. HLA-A2 antibody or antigen binding fragment thereof (or a fusion protein comprising the same and an endogenous CD3 epsilon protein) to HLA2 antigen at greater binding affinity than background binding. The term "directed against" is also applicable when considering the term "specificity" in understanding the interaction between antibody and epitope.

The term "antigen binding domain", refers to an antibody or antibody fragment that binds an antigen such as an HLA2 antigen. Antibodies or antibody fragments of the invention therefore include, but are not limited to polyclonal, monoclonal, bispecific, human, humanized or chimeric antibodies, single chain fragments (scFv), single variable fragments (ssFv), single domain antibodies (such as VHH fragments from nanobodies), Fab fragments, F(ab')2 fragments, fragments produced by a Fab expression library, anti-idiotypic antibodies and epitope-binding fragments or combinations thereof of any of the above, provided that they retain similar binding properties. Also mini-antibodies and multivalent antibodies such as diabodies, triabodies, tetravalent antibodies and peptabodies can be according to the present invention. The immunoglobulin molecules of the invention can be of any class (i.e., IgG, IgE, IgM, IgD and IgA) or subclass of immunoglobulin molecules. Thus, the term antibody, as used herein, also includes antibodies and antibody fragments either produced by the modification of whole antibodies or synthesized de novo using recombinant DNA methodologies. An antigen binding domain may in embodiments be an extracellular domain of a receptor, such as an endogenous receptor (e.g., if targeting CD137, CD137L may be added to CD3e for redirection).

As used herein, an "antibody" generally refers to a protein consisting of one or more polypeptides substantially encoded by immunoglobulin genes or fragments of immunoglobulin genes. Where the term "antibody" is used, the term "antibody fragment" may also be considered to be referred to. The recognized immunoglobulin genes include the kappa, lambda, alpha, gamma, delta, epsilon and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD, and IgE, respectively. The basic immunoglobulin (antibody) structural unit is known to comprise a tetramer or dimer. Each tetramer is composed of two identical pairs of polypeptide chains, each pair having one "light" (L) (about 25 kD) and one "heavy" (H) chain (about 50-70 kD). The N-terminus of each chain defines a variable region of about 100 to 1 10 or more amino acids, primarily responsible for antigen recognition. The terms "variable light chain" and "variable heavy chain" refer to these variable regions of the light and heavy chains respectively. A variable region of an antibody refers to the variable region of the antibody light chain or the variable region of the antibody heavy chain, either alone or in combination. The variable regions of the heavy and light chain each consist of four framework regions (FR) connected by three complementarity determining regions (CDRs) also known as hypervariable regions. The CDRs in each chain are held together in close proximity by the FRs and, with the CDRs from the other chain, contribute to the formation of the antigen-binding site of antibodies. Optionally, the antibody or the immunological portion of the antibody, can be chemically conjugated to, or expressed as, a fusion protein with other proteins.

"Single-chain Fv" or "scFv" antibody fragments comprise the VH and VL domains of an antibody, wherein these domains are present in a single polypeptide chain and in either orientation {e.g., VL- VH or VH-VL). Generally, a scFv polypeptide further comprises a polypeptide linker between the VH and VL domains which enables the scFv to form the desired structure for antigen binding. In preferred embodiments, the antigen binding domain within the in-frame fusion protein according to the present invention is an scFv antibody fragment that may be a murine, human or humanized scFv. Single chain antibodies may be cloned from the V region genes of a hybridoma specific for a desired target. The antigen binding domains and in-frame fusion proteins of the present invention comprising the same and an endogenous CD3 epsilon protein are intended to bind against mammalian, in particular human, protein targets. In particular embodiments, the antigen binding domain that is a humanized scFv that binds an autoantigen, alloantigen or cancer antigen. In particular embodiments the autoantigen or alloantigen is an HLA antigen, preferably HLA-A2. In further embodiments the cancer antigen is a ErbB protein or B-lymphocyte antigen, preferably HER2/neu or CD19.

In particular embodiments, the antigen-specific binding domain that is a humanized scFv that binds an HLA-A2 polypeptide. An illustrative example of a variable heavy chain that is suitable for constructing an in-frame fusion protein comprising an antigen binding domain specific for HLA-A2 contemplated herein include, but are not limited to the amino acid sequence set forth in SEQ ID NO: 47. An illustrative example of a variable light chain that is suitable for constructing anti- an in-frame fusion protein comprising an antigen binding domain specific for HLA-A2 contemplated herein include, but is not limited to the amino acid sequence set forth in SEQ ID NO: 47.

Affinities of the antigen binding domain and in-frame fusion proteins to specific antigens according to the present invention can be readily determined using conventional techniques, e.g., by competitive ELISA (enzyme-linked immunosorbent assay), or by binding association, or displacement assays using labeled ligands, or using a surface-plasmon resonance device such as the Biacore.

A "gene" refers to a DNA region that encodes a gene product including regions regulating the production of the gene product, regardless of whether these sequences are adjacent to coding and/or transcribed sequences. A gene comprises, but is not limited to, promoter sequences, terminators, translational regulatory sequences such as ribosome binding sites and internal ribosome entry sites, enhancers, silencers, insulators, boundary elements, replication origins, matrix attachment sites, and locus control regions.

The term "exon" refers to a part of a eukaryotic gene that is retained after splicing of the initially transcribed pre-mRNA. In contrast, the introns are cut out and degraded during splicing resulting in mature mRNA, which is transcribed into a protein sequence. The exons of protein-coding genes contain the open reading frame (ORF) and additionally the 5' and 3' untranslated region (UTR) from the terminal exons. The ORF is the region of DNA located between a start codon and a stop codon (reading frame). In embodiments the nucleic acid construct of the present invention is integrated into an exon of an endogenous CD3 epsilon gene. In preferred embodiments the nucleic acid construct of the present invention is integrated into exon 3 or exon 6 of an endogenous CD3 epsilon gene.

Gene editing is a type of genetic engineering in which DNA is inserted, deleted or replaced in the genome of a living organism by manipulated nucleases, or "molecular scissors". These nucleases generate site-specific double-strand breaks (DSBs) at desired sites in the genome. The induced double-strand breaks are repaired by non-homologous end compound (HEJ) or homologous recombination (HR), resulting in targeted mutations ("edits"). Five examples of manipulated nucleases can be used for gene editing: (a) meganucleases, (b) zinc finger nuclei (ZFNs), (c) transcription activator-like effector-based nucleases (TALENs), (d) Mega-TALENs, and (e) the CRISPR-Cas system.

Methods for introducing exogenous molecules, e.g., nucleic acid sequences, into cells are well known to experts and include lipid-mediated transfer (i.e. liposomes, including neutral and cationic lipids), electroporation, direct injection, cell fusion, particle bombardment, biopolymer nanoparticles, calcium phosphate co-precipitation, DEAE-dextran-mediated transfer and viral vector-mediated transfer. "Gene transfer", "nucleic acid transfer", gene sequence transfer", "transgene transfer" are used interchangeably. In embodiments, nucleic acid constructs are preferably transferred into cells by non-viral vectors and methods such as electroporation.

"Gene expression" is particularly the conversion of the information encoded by a gene into a gene product. A gene product can be the direct transcription product of a gene (e.g., mRNA, tRNA, rRNA, antisense RNA, ribozyme, structural RNA or any other type of RNA) or a protein produced by translation of a mRNA. Gene products also include RNAs modified by processes such as capping, polyadenylation, methylation, and editing, as well as proteins modified by processes such as methylation, acetylation, phosphorylation, ubiquitination, ADP-ribosylation, myristoylation, and glycosylation.

"Endogenous" as used herein is particularly used to define a gene expression that originates from a system such as a host, organism, tissue, or cell whereas "exogenous" particularly defines a genetic sequence introduced from externally into a cell by one or more genetic, biochemical or other methods.

The term "promoter" is used for a DNA sequence that initiates and regulates transcription from the DNA downstream of by binding of enzymes reading and transcribing the DNA. The resulting transcribed RNA may encode a protein. Promoters are preferably located near the transcription start sites of genes, upstream of the coding DNA, typically before the beginning of the open-reading frame (towards the 5' region of the sense strand).

In aspects of the invention, a method is provided in which the regulation of an integrated nucleic acid construct is placed under an endogenous promoter, whereby the nucleic acid construct to be integrated is preferably promoter-deficient. In some embodiments, the nucleic acid construct to be integrated, is preferably promoter-deficient. One such construct enables the integration of the nucleic acid construct, encoding an antigen binding domain, into a location within the genome such that the integrated nucleic acid sequence, is under the control of an endogenous promoter. In some embodiments, the nucleic acid construct is integrated into an endogenous CD3 epsilon gene. In some embodiments, the said endogenous promoter is a promoter of an endogenous CD3 epsilon gene.

An "integrated sequence", "integrating sequence" or "integrated nucleic acid construct" particularly refers to a nucleic acid construct including coding and/or non-coding elements to be inserted into the genome of the transfected cell also termed "host genome". In some embodiments, an edited chromosomal sequence may comprise an integrated sequence. The integrated sequence may code for an endogenous protein, an exogenous or heterologous protein, a wild-type protein, a modified protein, a fusion protein or similar. In embodiments of the present invention the integrated sequence encodes for an antigen binding domain.

An "integration site" or "fusion site" as used herein is the exact nucleic acid position within a genome where a double-strand break is generated and a nucleic acid construct is incorporated into the genome by homology directed repair at the position of the double-strand break. An endogenous sequence can be interrupted by integrating an exogenous sequence, so that the exogenous sequence is under the control of the endogenous promoter. In some embodiments the integrated exogenous sequence and/or the integrated exogenous sequence would be expressed together with an endogenous sequence. An integrated protein coding sequence can also be placed under the control of an endogenous promoter and/or fused in-frame with an endogenous protein coding sequence. In addition, the integrated sequence can also serve as a control element. Accordingly, the integrated sequence may be endogenous or exogenous to the cell. The exogenous sequence may be a homologue of or related to an endogenous sequence. The exogenous sequence can also be a human sequence. An animal or cell with such an integrated sequence can be called a "knock-in". In aspects of the invention, an integrated sequence can be a nucleic acid sequence encoding an antigen binding domain.

"Upstream" and "downstream" as used herein, are employed in the context of the 5'→3' direction of a nucleic acid strand. Upstream refers to nucleotides towards the 5' end and downstream towards the 3' end of any given nucleic acid.

A fusion product is defined as 'in-frame' when an open reading frame remains intact in the 3'-region downstream of the nucleic acid integration site, regardless of the number of inserted or deleted amino acids at the fusion junction point. A shift in the open reading frame of a protein coding gene sequence refers to "out-of-frame". According to the present invention the nucleic acid construct encoding for an antigen binding domain is preferably integrated in-frame into an endogenous CD3 epsilon gene.

According to the present invention the nucleic acid construct configured to be integrated in-frame with an endogenous CD3 epsilon gene of a human cell preferably comprises a first sequence encoding for an antigen binding domain, a second sequence encoding for a targeting sequence such as a left and/or a right homology arm, one or more sequences encoding for a linker and optionally a sequence encoding for a CD3 signal peptide, a protein separation site such as a self-cleavage peptide and/or an expression termination signal.

A "targeting sequence", "target sequence" or "target site" mainly refers to a chromosomal or extrachromosomal nucleic acid sequence that defines a part of a nucleic acid to which a binding molecule binds and/or cleaves, provided there are sufficient conditions for binding and/or cleavage. In gene editing approaches inducing double-strand breaks, the target sequence comprises an identical sequence to a recognition site of the endogenous DNA sequence into which an integration is intended, preferably selected from the group consisting of a homology arm, a guide RNA target site, a restriction enzyme recognition site, a ZFN recognition site, a ZFN cleaving site, a TALEN DNA-binding site, a recombinase recognition site, an integrase site and/or a homing nuclease recognition site. Techniques using each of these artificial nucleases are well known among experts. For example, when cells are edited with a genome editing complex (e.g., a TALEN, CRISPR-Cas9 derived from *Streptococcus pyogenes,* CRISPR/Cas12a derived from *Acidaminococcus* species BV3L6, ZFN, Mega-TALENs, or meganucleases to introduce a functional gene or to knock out a gene, nucleases (e.g., Cas9) induce a double strand break at the site of modification. Upon induction of the double strand break, a protein, such as a DNA repair protein, e.g., phosphorylated (serl778) 53BP1 (p53BPI) or gH2AC can accumulate at the site of the double strand break and is indicative of a DNA damage response.

The term "signal sequence" refers to a nucleic acid sequence encoding for a "signal peptide" also termed "target signal". A signal peptide is a short peptide usually comprising 16-30 amino acids present at the N-terminus of most synthesized proteins. The proteins comprising a signal peptide usually include proteins that reside either inside certain organelles (the endoplasmic reticulum, Golgi or endosomes), are secreted from the cell, or are inserted into cellular membranes such as the TCR/CD3 complex. At the end of the signal peptide there is typically a stretch of amino acids that is recognized and cleaved by signal peptidase and therefore named cleavage site. Signal peptidase may cleave either during or after completion of translocation to generate a free signal peptide and a mature protein.

The term "protein separation site", as used herein, comprise nucleic acid sequence elements that trigger separation of a bi-cistronic or a multi-cistronic expression of protein sequences in order to obtain distinctively expressed proteins. A protein separation site can, without limitation, refer to a self-cleavage peptide, an internal ribosome entry site (IRES), and/or a proteolytic cleavage site. In a preferred aspect of the invention, the nucleic acid construct can optionally be designed to include a protein separation site, preferably upstream of the nucleic acid sequences encoding an antigen binding domain.

A "self-cleavage peptide" or "self-cleaving peptide", e.g. P2A, can mediate ribosomal skipping during protein translation in a cell. The term "proteolytic cleavage site" refers to the recognition nucleic acid sequence for an intracellular proteolytic cleavage enzyme breaking the peptide bonds between amino acids in proteins.

In certain embodiments, the in-frame fusion proteins contemplated herein may comprise linker residues also termed "linker" between the various domains, added for appropriate spacing and conformation of the molecule, for example a linker comprising an amino acid sequence that connects the VH and VL domains and provides a spacer function compatible with interaction of the two sub-binding domains so that the resulting polypeptide retains a specific binding affinity to the same target molecule as an antibody that comprises the same light and heavy chain variable regions. In-frame fusion proteins contemplated herein, may comprise one, two, three, four, or five or more linkers. In particular embodiments, the length of a linker is about 1 to about 60 amino acids, about 5 to about 55 amino acids, or about 10 to about 50 amino acids, or any intervening length of amino acids such as 2, 4, 5, 6, 8, 10, 12, 14, 15, 16, 18, 20, 22, 24, 25, 26, 28, 30, 32, 34, 35, 36, 38, 40 42, 44, 45, 46 or 48 amino acids. Suitable linkers according to the present inventio include glycine peptides; glycine-serine peptides such as poly-Glycine-Serine (G4S); glycine-alanine peptides; alanine-serine peptides; and other flexible linkers known in the art, such as the Whitlow linker. Glycine and glycine-serine peptides are relatively unstructured, and therefore may be able to serve as a neutral tether between domains of fusion proteins such as the in-frame fusion proteins described herein. In particular embodiments, the antigen binding domain of the in-frame fusion protein is followed by one or more "spacers" or "spacer polypeptides," which refers to the region that moves the antigen binding domain away from the effector cell surface to enable proper cell/cell contact, antigen binding and activation.

An "Expression termination signal" comprises any structural elements at RNA and/or DNA level that specifies an end of transcription and/or translation, and/or a polyadenylation of the heterologous nucleic acid transcripts. The transcriptional stop is usually defined by a terminator, a nucleic acid sequence that defines the end of a transcription (e.g., of a gene) and initiates the release of the newly synthesized RNA. Terminators are located downstream of the gene to be transcribed and occur directly after 3' regulatory elements, such as the poly-A signal. The term "poly-A site", "poly-A signal" or "poly-A sequence", as used herein, refers to a nucleic acid sequence that signals both, termination and polyadenylation, of the nascent RNA transcript by RNA polymerase II. Polyadenylation sequences can enhance mRNA stability by adding a poly-A tail at the 3' end of the coding sequence, thus contributing to increased translation efficiency. Efficient polyadenylation of the recombinant transcript is preferable because transcripts without a poly-A tail are unstable and degrade rapidly. A "stop codon" is also a sequence of three consecutive nucleotides (termed triplet) within the messenger RNA that signals the termination of a protein translation process at the ribosome.

Polynucleotides such as the nucleic acid constructs according to the present invention can be prepared, manipulated and/or expressed using any of a variety of well- established techniques known and available in the art. In order to express a desired polypeptide, a nucleotide sequence encoding the polypeptide, can be inserted into appropriate vector. Examples of vectors are plasmid, autonomously replicating sequences, and transposable elements. Additional exemplary vectors include, without limitation, plasmids, phagemids, cosmids, artificial chromosomes such as yeast artificial chromosome (YAC), bacterial artificial chromosome (BAC), or PI-derived artificial chromosome (PAC), bacteriophages such as lambda phage or MI 3 phage, and animal viruses. Examples of categories of animal viruses useful as vectors include, without limitation, retrovirus (including lentivirus), adenovirus, adeno-associated virus, herpesvirus {e.g., herpes simplex virus), poxvirus, baculovirus, papillomavirus, and papovavirus {e.g., SV40). Examples of expression vectors are pClneo vectors (Promega) for expression in mammalian cells; pLenti4/V5-DEST^{™}, pLenti6/V5-DEST^{™}, and pLenti6.2/V5-GW/IacZ (Invitrogen) for lentivirus-mediated gene transfer and expression in mammalian cells. The "control elements" or "regulatory sequences" present in an expression vector are those non-translated regions of the vector - origin of replication, selection cassettes, promoters, enhancers, translation initiation signals (Shine Dalgarno sequence or Kozak sequence) introns, a polyadenylation sequence, 5' and 3' untranslated regions - which interact with host cellular proteins to carry out transcription and translation. Such elements may vary in their strength and specificity. Depending on the vector system and host utilized, any number of suitable transcription and translation elements, including ubiquitous promoters and inducible promoters may be used.

Methods for amplification of the nucleic acid constructs of the present invention are known to a person skilled in the art and include polymerase chain reaction (PCR)-based methods such as real-time PCR, quantitative real-time PCR (qPCR), quantitative real time reverse transcription PCR (qRT-PCR) and digital PCR using suitable primers hybridizing with the nucleic acid constructs.

The term "primer" refers to an oligonucleotide whether occurring naturally as in a purified restriction digest or produced synthetically, which is capable of acting as a point of initiation of synthesis when placed under conditions in which synthesis of a primer extension product which is complementary to a nucleic acid strand is induced, i.e., in the presence of nucleotides and an inducing agent such as DNA polymerase and at a suitable temperature and pH value. A primer thus can serve as a starting point for DNA-replicating enzymes such as DNA polymerase. The primer or oligonucleotide is preferably single stranded for maximum efficiency in amplification but may alternatively be double stranded. If double stranded, the primer is first treated to separate its strands before being used to prepare extension products. Preferably, the primer is an oligonucleotide, more preferably an oligodeoxyribonucleotide. The primer or oligonucleotide must be sufficiently long to prime the synthesis of extension products in the presence of the inducing agent. The exact lengths of the primers will depend on many factors, including temperature, source of primer and use of the method. For example, for diagnostics applications, depending on the complexity of the target sequence, the oligonucleotide primer typically contains 15-25 or more nucleotides, although it may contain fewer nucleotides. In embodiments of the invention primers according to SEQ ID NOs 43 to 46 are used for amplification of a nucleic acid construct according to the present invention encoding for an antigen binding domain to be integrated into an endogenous CD3 epsilon gene.

Hybridization is the process of establishing a non-covalent, sequence-specific interaction between two or more complementary strands of nucleic acids into a single hybrid, which in the case of two strands is referred to as a duplex or DNA double strand in the case of DNA. The terms "binding" or "annealing" may be used in place of hybridization in the present invention. The hybrids may be dissociated by thermal denaturation, also referred to as melting. Here, the solution of hybrids is heated to break the hydrogen bonds between nucleic bases, after which the two strands separate. In the absence of external negative factors, the processes of hybridization and melting may be repeated in succession indefinitely, which lays the ground for PCR.

As used herein, the term "hybridization" is used in reference to the pairing of complementary nucleic acids. Hybridization and the strength of hybridization (e.g., the strength of the association between the nucleic acids) is impacted by such factors as the degree of complementary between the nucleic acids, stringency of the conditions involved, the Tm (melting temperature) of the formed hybrid, and the G:C ratio within the nucleic acids. As used herein the term "stringency" is used in reference to the conditions of temperature, ionic strength, and the presence of other compounds such as organic solvents, under which nucleic acid hybridizations are conducted. In an illustrative example, hybridization under "highly stringent condition" can mean hybridization at 65 °C in 5X SSPE and 50% formamide and washing at 65 °C in 0.5X SSPE. In another illustrative example,"highly stringent condition" can mean hybridization at 55°C in a hybridization buffer consisting of 50% formamide (vol/vol); 10% dextran sulfate; 1 × Denhard"s solution; 20 mM sodium phosphate, pH 6.5; 5 × SSC; and 200 µg of salmon sperm DNA per ml of hybridization buffer for 18 to 24 hours, and washing four times (5 min each time) with 2 × SSC; 1% SDS at room temperature and then washing for 15 min at 50- 55°C with 0.1 × SSC. In another illustrative example Conditions for high stringency hybridization are described in Sambrook et al., "Molecular Cloning: A Laboratory Manual", 3rd Edition, Cold Spring Harbor Laboratory Press, (2001), incorporated herein by reference. In some illustrative aspects, hybridization occurs along the full-length of the nucleic acid. Detection of highly stringent hybridization in the context of the present invention indicates strong structural similarity or structural homology (e.g., nucleotide structure, base composition, arrangement or order) to, e.g., the nucleic acids provided herein.

The terms "Peptide" "polypeptide", "polypeptide fragment" and "protein" are used interchangeably, unless specified to the contrary, and according to conventional meaning, i.e., as a sequence of amino acids. Polypeptides are not limited to a specific length, e.g., they may comprise a full-length protein sequence or a fragment of a full-length protein, and may include post-translational modifications of the polypeptide, for example, glycosylations, acetylations, phosphorylations and the like, as well as other modifications known in the art, both naturally occurring and non-naturally occurring.

"Fusion proteins" or "chimeric proteins" as used herein, comprise proteins formed by joining two or more nucleic acid segments that originally encoded separate proteins, polypeptides or protein fragments. "Fusion gene", "gene fusion" or "fused gene" are according to the invention preferably joined nucleic acid sequence segments, wherein at least one nucleic acid sequence is also an exogenous nucleic acid sequence and at least one other nucleic acid sequence is also an endogenous nucleic acid sequence. In preferred embodiments, the exogenous nucleic acid is integrated into and fused to an endogenous nucleic acid sequence, e.g., an endogenous CD3 epsilon gene. In some embodiments, the integrated exogenous nucleic acid sequence to be fused may have several combined gene structures and/or polypeptide-encoding sequences separated by one or more protein separation sites. The translation of a fusion gene results in a single or multiple polypeptides with functional properties derived from each of the original proteins or protein fragments. In a further embodiment, the in-frame fusion proteins contemplated herein comprise a signal (or leader) sequence at the N-terminal end of the protein, which co-translationally or post-translationally directs transfer of the protein. Polypeptides can be prepared using any of a variety of well-known recombinant and/or synthetic techniques. Polypeptides contemplated herein specifically encompass the in-frame fusion proteins of the present disclosure, or sequences that have deletions from, additions to, and/or substitutions of one or more amino acid of an in-frame fusion protein as disclosed herein.

An "isolated peptide" or an "isolated polypeptide" and the like, as used herein, refer to in vitro isolation and/or purification of a peptide or polypeptide molecule from a cellular environment, and from association with other components of the cell, i.e., it is not significantly associated with in vivo substances. Similarly, an "isolated cell" refers to a cell that has been obtained from an in vivo tissue or organ and is substantially free of extracellular matrix.

The present invention contemplates, in particular embodiments, T cells genetically modified to express the in-frame fusion protein contemplated herein, for use in the prevention and/or treatment of an unwanted (pathogenic) immune reaction such as an unwanted (pathogenic) immune reaction prior to and/or after an allogenic transplantation. In preferred embodiments the invention contemplates T cells genetically modified to express the in-frame fusion protein contemplated herein, for use in the prevention and/or treatment of graft-versus host disease. In further embodiments the invention contemplates T cells genetically modified to express the in-frame fusion protein contemplated herein, for use in the prevention and/or treatment of cancerous diseases.

As used herein, the term "genetically engineered" or "genetically modified" refers to the addition of extra genetic material in the form of DNA or RNA into the total genetic material in a cell. In contrast an "unmodified" cell relates to a cell where no extra genetic material added or introduced into the total genetic material of the cell. Genetic modifications can be carried out selectively or not at a specific location in the genome of a cell. In an embodiment, the genetic change is site-specific. In an embodiment, the genetic modification is not site-specific. The terms, "genetically modified cells"," genetically modified immune cells", "modified cells," and, "redirected cells," are used interchangeably. As used herein, the term "gene therapy" refers to the introduction-permanently or transiently- of extra genetic material in the form of DNA or RNA into the total genetic material in a cell that restores, corrects, or modifies expression of a gene, or for the purpose of expressing a polypeptide, e.g., an antigen binding domain. In particular embodiments, the antigen binding domains contemplated herein are introduced and expressed as an in-frame fusion protein comprising the antigen binding domain and an endogenous CD3 epsilon protein in a T cell so as to direct their specificity to a target antigen of interest, e.g., a HLA-A2 antigen.

T cells of the invention can be autologous/autogeneic ("self") or non-autologous ("non- self," e.g., allogeneic, syngeneic or xenogeneic). "Autologous", as used herein, refers to cells from the same subject, and represent a preferred embodiment of the invention. "Allogeneic," as used herein, refers to cells of the same species that differ genetically to the cell in comparison. "Syngeneic," as used herein, refers to cells of a different subject that are genetically identical to the cell in comparison. "Xenogeneic," as used herein, refers to cells of a different species to the cell in comparison. In preferred embodiments, the cells of the invention are autologous or allogeneic.

In particular embodiments, prior to in vitro manipulation or genetic modification of the immune effector cells described herein, the source of cells is obtained from a subject. T cells can be obtained from a number of sources including, but not limited to, peripheral blood mononuclear cells, bone marrow, lymph nodes tissue, cord blood, thymus issue, tissue from a site of infection, ascites, pleural effusion, spleen tissue, and tumors. In certain embodiments, T cells can be obtained from a unit of blood collected from a subject using any number of techniques known to the skilled person, such as sedimentation, e.g., FICOLL^{™} separation, antibody-conjugated bead-based methods such as MACS^{™} separation (Miltenyi). In one embodiment, cells from the circulating blood of an individual are obtained by apheresis. The apheresis product typically contains lymphocytes, including T cells, monocytes, granulocyte, B cells, other nucleated white blood cells, red blood cells, and platelets. In one embodiment, the cells collected by apheresis may be washed to remove the plasma fraction and to place the cells in an appropriate buffer or media for subsequent processing. The cells can be washed with PBS or with another suitable solution that lacks calcium, magnesium, and most, if not all other, divalent cations. As would be appreciated by those of ordinary skill in the art, a washing step may be accomplished by methods known to those in the art, such as by using a semiautomated flow through centrifuge. For example, the Cobe 2991 cell processor, the Baxter CytoMate, or the like. After washing, the cells may be resuspended in a variety of biocompatible buffers or other saline solution with or without buffer. In certain embodiments, the undesirable components of the apheresis sample may be removed in the cell directly resuspended culture media.

In certain embodiments, T cells are isolated from peripheral blood mononuclear cells (PBMCs) by lysing the red blood cells and depleting the monocytes, for example, by centrifugation through a PERCOLL^{™} gradient. A specific subpopulation of T cells can be further isolated by positive or negative selection techniques. One method for use herein is cell sorting and/or selection via negative magnetic immunoadherence or flow cytometry that uses a cocktail of monoclonal antibodies directed to cell surface markers present on the cells negatively selected.

The T cells can be genetically modified following isolation and optionally sorting using known methods, or the T cells can be activated and expanded (or differentiated in the case of progenitors) in vitro prior to being genetically modified. In a particular embodiment, T cells, such as Tregs, are genetically modified with the nucleic acid construct comprising a sequence region encoding for an antigen binding domain as contemplated herein (e.g., transfected with a nucleic acid construct and pre-complexed Cas12a enzyme or Cas9 enzyme) and then are expanded in vitro. In various embodiments, T cells can be activated and expanded before or after genetic modification to express the in-frame fusion protein, using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005. For example, expansion of Treg cells requires repetitive stimulation via their TCR/CD3 complex and additional CD28 co-stimulation using anti-CD3 and anti-CD28 antibodies on beads. A high dose of IL-2 as a growth factor and the mTOR inhibitor rapamycin are also added to the medium. In one aspect the invention relates to a method of preparing a genetically modified T cell, comprising repetitive stimulation via the TCR/CD3 complex, whereby the genetically modified T cell exhibits unmodified expansion dynamics in culture (+/- 50%) in comparison to an unmodified T cell. In one embodiment of the present invention the genetically modified T cells can be by said method to an unmodified extent (+/- 50%) in comparison to an unmodified T cell.

In one embodiment, the invention provides a method of storing the genetically modified T cells which target e.g., an HLA-A2 antigen, comprising cryopreserving the T cells such that the cells remain viable upon thawing. A fraction of the genetically modified T cells expressing can be cryopreserved by methods known in the art to provide a permanent source of such cells for the future administration to a subject, such as for the prevention and/or treatment of an unwanted (pathogenic) immune reaction or a cancerous disease. When needed, the cryopreserved transformed T cells can be thawed, grown and expanded for more such cells.

A "pharmaceutical composition" refers to a composition formulated in pharmaceutically-acceptable or physiologically- acceptable solutions for administration to a cell or an animal, either alone, or in combination with one or more other modalities of therapy. It will also be understood that, if desired, the compositions of the invention may be administered in combination with other agents as well, such as, e.g., cytokines, growth factors, hormones, small molecules, chemotherapeutics, pro-drugs, drugs, antibodies, or other various pharmaceutically-active agents. There is virtually no limit to other components that may also be included in the compositions, provided that the additional agents do not adversely affect the ability of the composition to deliver the intended therapy.

The term "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

As used herein "pharmaceutically acceptable carrier, diluent or excipient" includes without limitation any adjuvant, carrier, excipient, glidant, sweetening agent, diluent, preservative, dye/colorant, flavor enhancer, surfactant, wetting agent, dispersing agent, suspending agent, stabilizer, isotonic agent, solvent, surfactant, or emulsifier which has been approved by the United States Food and Drug Administration as being acceptable for use in humans or domestic animals.

Plasmids, nucleic acids, nucleic acids complexed with a Cas enzyme, and/or other compositions, agents, drugs, biologics (proteins) can be incorporated into pharmaceutical compositions, e.g., pharmaceutically acceptable carriers or excipients. Such pharmaceutical compositions are particularly useful for administration and delivery to a subject in vivo or ex vivo.

Pharmaceutical compositions of the present invention comprising a genetically modified T cell population, such as genetically modified Treg cells, may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions of the present invention are preferably formulated for parenteral administration, e.g., intravascular (intravenous or intraarterial), intraperitoneal or intramuscular administration.

The liquid pharmaceutical compositions, whether they are solutions, suspensions or other like form, may include one or more of the following: sterile diluents such as water for injection, saline solution, preferably physiological saline, Ringer's solution, isotonic sodium chloride, fixed oils such as synthetic mono or diglycerides which may serve as the solvent or suspending medium, polyethylene glycols, glycerin, propylene glycol or other solvents; antibacterial agents such as benzyl alcohol or methyl paraben; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic. An injectable pharmaceutical composition is preferably sterile.

In particular embodiments, pharmaceutical compositions contemplated herein comprise an amount of genetically modified T cells, in combination with one or more pharmaceutically or physiologically acceptable carriers, diluents or excipients. In particular embodiments, pharmaceutical compositions contemplated herein comprise an amount of genetically modified genetically modified T cells in combination with one or more pharmaceutically or physiologically acceptable carriers, diluents or excipients.

As used herein "treatment" or "treating," includes any beneficial or desirable effect on the symptoms or pathology of a disease or pathological condition, and may include even minimal reductions in one or more measurable markers of the disease or condition being treated. Treatment can involve optionally either the reduction or amelioration of symptoms of the disease or condition, or the delaying of the progression of the disease or condition. "Treatment" does not necessarily indicate complete eradication or cure of the disease or condition, or associated symptoms thereof.

As used herein, "prevent," and similar words such as "prevented," "preventing" or "prophylactic" etc., indicate an approach for preventing, inhibiting, or reducing the likelihood of the occurrence or recurrence of, a disease or condition. It also refers to delaying the onset or recurrence of a disease or condition or delaying the occurrence or recurrence of the symptoms of a disease or condition. As used herein, "prevention" and similar words also includes reducing the intensity, effect, symptoms and/or burden of a disease or condition prior to onset or recurrence of the disease or condition.

In one embodiment, a method of preventing and/or treating a unwanted (pathogenic) immune reaction or cancerous disease in a subject comprises administering an effective amount, e.g., therapeutically effective amount of a composition comprising genetically modified immune effector cells contemplated herein. The quantity and frequency of administration will be determined by such factors as the condition of the patient, and the type and severity of the patient's disease, although appropriate dosages may be determined by clinical trials.

The administration of the compositions contemplated herein may be carried out in any convenient manner, including by aerosol inhalation, injection, ingestion, transfusion, implantation or transplantation. In a preferred embodiment, compositions are administered parenterally. The phrases "parenteral administration" and "administered parenterally" as used herein refers to modes of administration other than enteral and topical administration, usually by injection, and includes, without limitation, intravascular, intravenous, intramuscular, intraarterial, intrathecal, intracapsular, intraorbital, intratumoral, intracardiac, intradermal, intraperitoneal, transtracheal, subcutaneous, subcuticular, intraarticular, subcapsular, subarachnoid, intraspinal and intrasternal injection and infusion.

In particular embodiments, compositions of the present invention comprise an amount of genetically modified T cells contemplated herein. As used herein, the term "amount" refers to "an amount effective" or "an effective amount" of a genetically modified therapeutic T cell, to achieve a beneficial or desired prophylactic or therapeutic result, including clinical results.

A "prophylactically effective amount" refers to an amount of a genetically modified T cell effective to achieve the desired prophylactic result. Typically but not necessarily, since a prophylactic dose is used in subjects prior to or at an earlier stage of an unwanted (pathogenic) immune reaction or cancerous disease, the prophylactically effective amount is less than the therapeutically effective amount. The term prophylactic does not necessarily refer to a complete prohibition or prevention of a particular medical condition such as an unwanted (pathogenic) immune reaction or cancerous disease. The term prophylactic also refers to the reduction of risk of a certain medical condition occurring or worsening in its symptoms.

A "therapeutically effective amount" of a genetically modified T cell may vary according to factors such as the disease state, age, sex, and weight of the individual. A therapeutically effective amount is also one in which any toxic or detrimental effects of the genetically modified cells are outweighed by the therapeutically beneficial effects. The term "therapeutically effective amount" includes an amount that is effective to "treat" a subject {e.g., a patient). When a therapeutic amount is indicated, the precise amount of the compositions of the present invention to be administered can be determined by a physician with consideration of individual differences in age, weight, stage of the medical condition, and condition of the patient (subject). It can generally be stated that a pharmaceutical composition comprising the genetically modified T cells described herein may be administered at a dosage of 10² to 10¹⁰ cells/kg body weight, preferably 10⁵ to 10⁷ cells/kg body weight, including all integer values within those ranges. The number of cells will depend upon the ultimate use for which the composition is intended as will the type of cells included therein. For uses provided herein, the cells are generally in a volume of a liter or less, can be 500 mL or less, even 250 mL or 100 mL or less. Hence the density of the desired cells is typically greater than 10⁶ cells/ml and generally is greater than 10⁷ cells/mL, generally 10⁸ cells/mL or greater. The clinically relevant number of T cells can be apportioned into multiple infusions that cumulatively equal or exceed 10⁵, 10⁶, 10⁷, 10⁸, 10⁹, 10¹⁰, 10¹¹, or 10¹² cells. In some aspects of the present invention, particularly since all the infused cells will be directed to a particular target antigen, lower numbers of cells may be administered. Compositions of genetically modified T cells may be administered multiple times at dosages within these ranges.

The cells may be allogeneic, syngeneic, xenogeneic, or autologous to the patient undergoing therapy.

Immunotherapy in the context of the present invention is to be understood to comprise any therapeutic agent employing the immune system for prevention and/or treatment of an unwanted (pathogenic) immune reaction such as an immune reaction prior to and/or after allogenic transplantation and an autoimmune disease and for treatment of cancerous diseases. With regards to cancer immunotherapy takes advantage of the fact that cancer cells have subtly different molecules on their surface, which can be recognized by the immune system. Immunotherapy encompasses, without limitation, cellular and antibody therapy. Cellular therapies according to the present invention involve the administration of genetically modified T cells expressing comprising and/or expressing an in-frame fusion protein comprising an antigen binding domain and an endogenous CD3 epsilon protein, whereby the TCR complex function is maintained.

In a particular embodiment, compositions contemplated herein comprise an effective amount of genetically modified T cells, alone or in combination with one or more therapeutic agents. Thus, the genetically modified T cell compositions may be administered alone or in combination with other known treatments for the respective medical condition such as immunomodulatory or cancer treatments, including immunotherapy, hormone therapy, photodynamic therapy, radiation therapy, chemotherapy, transplantation, etc. The compositions may also be administered in combination with antibiotics. Such therapeutic agents may be accepted in the art as a standard treatment for a particular disease state as described herein, such as a particular autoimmune disease or cancerous disease. Exemplary therapeutic agents contemplated include cytokines, growth factors, steroids, NSAIDs, DMARDs, anti-inflammatories, chemotherapeutics, radiotherapeutics, therapeutic antibodies, or other active and ancillary agents.

A combined immunotherapy encompasses simultaneous treatment, co-treatment or joint treatment, and includes the administration of genetically modified T cells combined with immunotherapies, such as checkpoint inhibitors and/or immune stimulatory cytokines, whereby treatment may occur within minutes of each other, in the same hour, on the same day, in the same week or in the same month as one another. A combination medicament, comprising one or more of said genetically modified T cells with another immunotherapeutic, may also be used in order to co-administer the various components in a single administration or dosage. A "medicament" particularly refers to a drug including an immune-therapeutic cell or genetically modified cell used to cure, treat, or prevent a disease.

A "Cancerous disease", as used herein, is a disease characterized by the uncontrolled growth of abnormal cells. Cancer refers to any type of cancerous growth or carcinogenic process, metastatic tissue or malignant transformed cells, tissues or organs, regardless of histopathological type or invasive stage. Cancer cells can spread locally or to other parts of the body via the bloodstream and lymphatic system. Cancer cells spreading to other parts to the body are termed "metastatic cells" or "metastatic tumor cells". The terms "tumor" and "cancer", used herein, are utilized interchangeably, e.g. both terms include solid and liquid, e.g. general or circulating tumors, premalignant and malignant cancers and tumors. Examples of liquid cancers include, but not limited to, acute lymphocytic leukemia (ALL), chronic lymphocytic leukemia (CLL), acute myeloid Leukemia (acute myelogenous leukemia (AML), chronic bone marrow cancer, chronic myelogenous leukemia (CML), Hodgkin lymphoma, non-Hodgkin lymphoma, and myeloma. Examples of solid tumors are liver, lung, breast, lymphatic system, digestive organs (e.g. colon), urogenital organs (e.g. kidney, urothelial cells), prostate and throat, malignant organ systems including tumors such as sarcomas, adenocarcinomas and cancer. Examples for breast cancer that can be treated with the are ductal carcinoma in situ (DCIS), lobular carcinoma in situ (LCIS), invasive ductal carcinoma (IDC), invasive ductal carcinoma including tubular, medullary, mucinous, papillary, and cribriform carcinomas, invasive lobular carcinoma (ILC), inflammatory breast cancer, male breast cancer, Paget's Disease of the nipple, phyllodes tumors of the breast, recurrent and/or metastatic breast cancer. Adenocarcinoma includes most malignant tumors such as colon cancer, rectal cancer, renal cell cancer, liver cancer, non-small cell lung cancer, small intestine cancer and esophageal cancer. In certain forms the cancer is a melanoma, e.g. an advanced stage melanoma. Metastatic lesions of the cancer can also be treated or prevented with the methods and compositions of the invention. Examples of other types of cancer that can be treated are bone cancer, pancreatic cancer, skin cancer, head and neck cancer, skin or intraocular melanoma, uterine cancer, ovarian cancer, rectal cancer, anal cancer, stomach cancer, testicular cancer, faropius duct cancer, Endometrial cancer, cervical cancer, vaginal cancer, vulvar cancer, Hodgkin's disease, non-Hodgkin's lymphoma, esophageal cancer, small intestine cancer, endocrine cancer, thyroid cancer, parathyroid cancer, adrenal cancer, soft tissue sarcoma, urethral cancer, penile cancer, acute myeloid leukemia, chronic myelocytic leukemia, acute lymphoblastic leukemia, chronic or acute leukemia including chronic lymphatic leukemia, solid tumor in childhood, lymphatic lymphoma, bladder cancer, kidney or ureter cancer, renal pelvis cancer, neoplasm of the central nervous system (CNS) primary CNS lymphoma, tumor angiogenesis, spinal axis tumor, brainstem glioma, pituitary adenoma, Kaposi's sarcoma, epidermoid carcinoma, squamous cell carcinoma, asbestos-induced T cell lymphoma Including a combination of environmental cancer and cancer including things Treatment of metastatic cancer, e.g. metastatic cancer that expresses PD-L1 (Iwai et al. (2005) Int. Immunol. 17: 133-144) can be performed with the inhibitory molecules described in this invention.

The medical indications associated with an unwanted (pathogenic) immune response represent preferred embodiments of the medical use of the invention in the treatment and/or prevention of a unwanted (pathogenic) immune response. Further, medical indications associated with a cancerous disease represent embodiments of the medical use of the invention in the treatment and/or prevention of a cancerous disease.

### SEQUENCES

**Table 1: guide RNA target on CD3 epsilon (inventive sequences) and TRAC (comparative example)**

| | | | | |
|---|---|---|---|---|
| Any of the sequences SEQ ID 1 to 8 provided in the table below, any combination of sequences, or any marked sub-regions of the sequence may represent an embodiment of the invention and/or be employed in any of the aspects or embodiments of the invention described herein. | | | | |

| **SEQ ID NO** | **Sequence 5'-3'** | **name** | **nuclease** | **target** |
|---|---|---|---|---|
| 1 | GGGGCAAGATGGTGAGATATGCT | gRNA1: AsCas12a crRNA ex3 #1 | AsCas 12a | CD3E exon 3 |
| 2 | TAGTTGGCGTTTGGGGGCAAGAT | gRNA2: AsCas12a crRNA ex3 #2 | AsCas 12a | CD3E exon 3 |
| 3 | TTTTCTAGTTGGCGTTTGGGGGC | gRNA3: AsCas12a crRNA ex3 #3 | AsCas 12a | CD3E exon 3 |
| 4 | TCCCCAGCATATAAAGTCTCCAT | gRNA4: AsCas12a crRNA ex6 #1 | AsCas 12a | CD3E exon 6 |
| 5 | AGATCCAGGATACTGAGGGCATG | gRNA5: AsCas12a crRNA ex6 #2 | AsCas 12a | CD3E exon 6 |
| 6 | AGGGCATGTCAATATTACTG | gRNA6: Cas9 ex6 #1 | SpCas9 | CD3E exon 6 |
| 7 | GATGAGGATGATAAAAACAT | gRNA7: Cas9 ex6 #2 | SpCas9 | CD3E exon 6 |
| 8 | GTTGGCGTTTGGGGGCAAGA | gRNA8: Cas9 #3 for CD3e ex3 | SpCas9 | CD3E exon 3 |
| 9 | GAGAATCAAAATCGGTGAAT | gRNA9: ourTRAC | SpCas9 | TRAC exon 1 |

**Table 2: nucleic acid sequence for HDR donor templates for CD3 epsilon (inventive sequences)**

| Any of the sequences SEQ ID 10 to 28 provided in the table below, any combination of sequences, or any marked sub-regions of the sequence may represent an embodiment of the invention and/or be employed in any of the aspects or embodiments of the invention described herein. | | | |
|---|---|---|---|
| **SEQ ID NO** | **Homology-Directed DNA Repair (HDR) Template** | **Info HDR-Template Part** | **Sequence 5'-3'** |
| 10 | HDRT HLA-A2 scFv into CD3e-exon3 | Full sequence | TACCCAACCAAGAGCGTGTTTTTCTTCCACAGACACCAATGT TCAAAATGGAGGCTTGGGGGCAAAATTCTTTTGCTATGTCTC TAGTCGTCCAAAAAATGGTCCTAACTTTTTCTGACTCCTGCTT GTCAAAAATTGTGGGCTCATAGTTAATGCTAGATGCTTCCTT CCTCTATTTCCCCCCAAATTTCCTGGGAACCCCTGGTCAATA CCAGCAGTAAGTTCCACTGTTCTAGGGTGTAGAAATGGCTG TGACCCAGCAGCAAGAGGGAAGGACATCAGATGTCATCAGT GGTCATACTGCAACACAGCCCTTTTTCTGTTTAGGAATGCAG GTACCCACAACATTTACTAACACTTTTTTTTTCTTATTTATTTT CTAGTTGGCGTTTGGGGGCAAGAGCAGAAGCTCATCAGTGA AGAAGATCTGCAAGTGCAACTGGTGCAGTCAGGAGGAGGA GTTGTGCAGCCAGGAGGAAGTCTGCGGGTCAGTTGCGCCG CGTCAGGCGTTACTCTCTCCGATTATGGAATGCATTGGGTCA GACAGGCACCAGGCAAAGGTCTGGAATGGATGGCATTCATT CGCAATGACGGGTCGGATAAGTATTACGCTGACAGCGTAAA GGGCAGGTTTACAATCTCTCGGGATAATAGCAAAAAAACTGT CAGCCTGCAGATGAGTTCACTGCGCGCCGAGGACACCGCT GTGTACTATTGTGCTAAAAACGGCGAGTCTGGGCCATTGGA |
| | | | TTATTGGTACTTTGATCTCTGGGGGCGGGGAACACTGGTGA CTGTGTCTGGAGGTGGTGGATCAGGAGGTGGAGGTTCCGG AGGTGGAGGAAGTGACGTCGTGATGACTCAGTCTCCCTCCT CTCTGAGTGCCAGCGTTGGGGATCGCGTTACGATCACCTGC CAAGCCAGCCAGGATATCAGCAACTACTTAAATTGGTATCAG CAAAAGCCTGGAAAGGCTCCAAAGCTGCTAATCTACGACGC GTCCAACCTGGAGACCGGTGTTCCATCACGCTTCTCTGGAT CTGGGTCTGGTACCGATTTTACATTCACCATATCTTCTTTGC AACCCGAGGACTTCGCTACCTATTATTGCCAACAGTATAGCA GCTTCCCTTTAACTTTCGGGGGAGGCACTAAAGTGGATATCA AGCGGGGAGGCGGAGGTAGCGGAGGCGGCGGAAGCGGTG GAGGTGGGTCTCAAGATGGTGAGATATGCTTTCTTTCTTTCT TTTTTATGAAATCACCCCATCATTCTTTGTAGTTATGAATGGA GCTTTCTCTTAGGCCTCCCACAGAACTTCCACAGAGGTCAG GAAAAGGAGTTTCTGCCATCTACCCCTTTGACTTTCCTCACA AGTCTGGAGATATTTCTAGCCCAGAAGAGGGAAGCAACAGA GGCAGGAAATAATGAGTCTTAACCATACAAAAGAAAAATTGA GACTTAAATGAAGTTGAAAGCACTAACAGTTTTCATTTGTTTG CATTTCATATTTGATGTGAGATTCTGCAGAGGAGACGTAGCC AGAATGCATGCACAGGGTTACTCTGGATAAGCTGCTGGGGC AACATTTGGATGTGTGTTCAGAATCACATGTCTGAAT |
| 11 | | Left homology arm | TACCCAACCAAGAGCGTGTTTTTCTTCCACAGACACCAATGT TCAAAATGGAGGCTTGGGGGCAAAATTCTTTTGCTATGTCTC TAGTCGTCCAAAAAATGGTCCTAACTTTTTCTGACTCCTGCTT GTCAAAAATTGTGGGCTCATAGTTAATGCTAGATGCTTCCTT CCTCTATTTCCCCCCAAATTTCCTGGGAACCCCTGGTCAATA CCAGCAGTAAGTTCCACTGTTCTAGGGTGTAGAAATGGCTG TGACCCAGCAGCAAGAGGGAAGGACATCAGATGTCATCAGT GGTCATACTGCAACACAGCCCTTTTTCTGTTTAGGAATGCAG GTACCCACAACATTTACTAACACTTTTTTTTTCTTATTTATTTT CTAGTTGGCGTTTGGGGGCAA |
| 12 | | Myc tag | GAGCAGAAGCTCATCAGTGAAGAAGATCTG |
| 13 | | hu anti-HLA-A2 VH (clone: 3PF12) | CAAGTGCAACTGGTGCAGTCAGGAGGAGGAGTTGTGCAGC CAGGAGGAAGTCTGCGGGTCAGTTGCGCCGCGTCAGGCGT TACTCTCTCCGATTATGGAATGCATTGGGTCAGACAGGCAC CAGGCAAAGGTCTGGAATGGATGGCATTCATTCGCAATGAC GGGTCGGATAAGTATTACGCTGACAGCGTAAAGGGCAGGTT TACAATCTCTCGGGATAATAGCAAAAAAACTGTCAGCCTGCA GATGAGTTCACTGCGCGCCGAGGACACCGCTGTGTACTATT GTGCTAAAAACGGCGAGTCTGGGCCATTGGATTATTGGTAC TTTGATCTCTGGGGGCGGGGAACACTGGTGACTGTGTCT |
| 14 | | optimized (G4S)3 linker | GGAGGTGGTGGATCAGGAGGTGGAGGTTCCGGAGGTGGAG GAAGT |
| 15 | | hu anti-HLA-A2 VL (clone: 3PF12) | GACGTCGTGATGACTCAGTCTCCCTCCTCTCTGAGTGCCAG CGTTGGGGATCGCGTTACGATCACCTGCCAAGCCAGCCAG GATATCAGCAACTACTTAAATTGGTATCAGCAAAAGCCTGGA AAGGCTCCAAAGCTGCTAATCTACGACGCGTCCAACCTGGA GACCGGTGTTCCATCACGCTTCTCTGGATCTGGGTCTGGTA CCGATTTTACATTCACCATATCTTCTTTGCAACCCGAGGACT TCGCTACCTATTATTGCCAACAGTATAGCAGCTTCCCTTTAA CTTTCGGGGGAGGCACTAAAGTGGATATCAAGCGG |
| 16 | | optimized (G4S)3 linker | GGAGGCGGAGGTAGCGGAGGCGGCGGAAGCGGTGGAGGT GGGTCT |
| 17 | | Right homology arm | CAAGATGGTGAGATATGCTTTCTTTCTTTCTTTTTTATGAAAT CACCCCATCATTCTTTGTAGTTATGAATGGAGCTTTCTCTTAG GCCTCCCACAGAACTTCCACAGAGGTCAGGAAAAGGAGTTT CTGCCATCTACCCCTTTGACTTTCCTCACAAGTCTGGAGATA TTTCTAGCCCAGAAGAGGGAAGCAACAGAGGCAGGAAATAA TGAGTCTTAACCATACAAAAGAAAAATTGAGACTTAAATGAA GTTGAAAGCACTAACAGTTTTCATTTGTTTGCATTTCATATTT |
| | | | GATGTGAGATTCTGCAGAGGAGACGTAGCCAGAATGCATGC ACAGGGTTACTCTGGATAAGCTGCTGGGGCAACATTTGGAT GTGTGTTCAGAATCACATGTCTGAAT |
| 18 | HDRT HLA-A2 scFv into CD3e-exon6 | Full Sequence | TCTTCCCAGCATTGCATTCTCAACTCCATTTTAGAAAGGTTC CAAATAGGGACTTCTGTGGGTTTTTCTTTACATCCATCTTACC CTTCCCAAGTCCCCATGTCCCTGCGTAAACCCTAAAGCCAC CTCTCAAAAGGTTCTCTAGTTCCCTTCAAGGTTCTCTAGTTC CCTTCATTCCACATATCTCCTCTTCCACACCCTCTAGCCAGT AGAGCTCCCTTCTGACAAGCAAGTCTAAGATCTAGATGACAG ATGACTTCCTGCATTTGGGTGGTTCTTTTGTCACTAATTTGC CTTTTCTAAAATTGTCCTGGTTTCTTCTGCCAATTTCCCTTCT TTCTCCCCAGCATATAAAGTCTCCATCTCTGGAACCACAGTA ATATTGACATGCCCTCAGTATGCCACCAACTTCTCTCTTTTG AAGCAGGCCGGAGATGTGGAAGAAAATCCTGGGCCCATGC AATCAGGCACTCACTGGCGAGTGCTGGGTCTGTGCTTGCTC AGCGTAGGAGTATGGGGTCAAGAGCAGAAGCTCATCAGTGA AGAAGATCTGCAAGTGCAACTGGTGCAGTCAGGAGGAGGA GTTGTGCAGCCAGGAGGAAGTCTGCGGGTCAGTTGCGCCG CGTCAGGCGTTACTCTCTCCGATTATGGAATGCATTGGGTCA GACAGGCACCAGGCAAAGGTCTGGAATGGATGGCATTCATT CGCAATGACGGGTCGGATAAGTATTACGCTGACAGCGTAAA GGGCAGGTTTACAATCTCTCGGGATAATAGCAAAAAAACTGT CAGCCTGCAGATGAGTTCACTGCGCGCCGAGGACACCGCT GTGTACTATTGTGCTAAAAACGGCGAGTCTGGGCCATTGGA TTATTGGTACTTTGATCTCTGGGGGCGGGGAACACTGGTGA CTGTGTCTGGAGGTGGTGGATCAGGAGGTGGAGGTTCCGG AGGTGGAGGAAGTGACGTCGTGATGACTCAGTCTCCCTCCT CTCTGAGTGCCAGCGTTGGGGATCGCGTTACGATCACCTGC CAAGCCAGCCAGGATATCAGCAACTACTTAAATTGGTATCAG CAAAAGCCTGGAAAGGCTCCAAAGCTGCTAATCTACGACGC GTCCAACCTGGAGACCGGTGTTCCATCACGCTTCTCTGGAT CTGGGTCTGGTACCGATTTTACATTCACCATATCTTCTTTGC AACCCGAGGACTTCGCTACCTATTATTGCCAACAGTATAGCA GCTTCCCTTTAACTTTCGGGGGAGGCACTAAAGTGGATATCA AGCGGGGAGGTGGTGGATCAGGAGGTGGAGGTTCCGGAG GTGGAGGAAGTGACGGTAACGAAGAGATGGGGGGAATCAC CCAGACACCATACAAAGTCAGCATCAGCGGCACAACGGTCA TCCTGACGTGTCCTCAATACCCTGGATCTGAAATACTATGGC AACACAATGATAAAAACATAGGCGGTGATGAGGATGATAAAA ACATAGGCAGTGATGAGGATCACCTGTCACTGAAGGAATTTT CAGAATTGGAGCAAAGTGGTTATTATGTCTGCTACCCCAGAG GAAGCAAACCAGAAGATGCGAACTTTTATCTCTACCTGAGG GCAAGAGGTAATCCAGGTCTCCAGAACAGGTACCACCGGCT CTTTAGGGAGGACCATTCAAAAGGGCATTCTCAGTGATTTTC CCTAACCCAGCTCACAGTGCCCAGGCGTCTTTGCGCTTCCT CCCACACTCAATCCTGGGACTCTCTGGTACCACACGGCATC AGTGTTTTCTGGAATATAGATTAAACACCAATATGAGGCTTCT GGG |
| 19 | | Left homology arm | TCTTCCCAGCATTGCATTCTCAACTCCATTTTAGAAAGGTTC CAAATAGGGACTTCTGTGGGTTTTTCTTTACATCCATCTTACC CTTCCCAAGTCCCCATGTCCCTGCGTAAACCCTAAAGCCAC CTCTCAAAAGGTTCTCTAGTTCCCTTCAAGGTTCTCTAGTTC CCTTCATTCCACATATCTCCTCTTCCACACCCTCTAGCCAGT AGAGCTCCCTTCTGACAAGCAAGTCTAAGATCTAGATGACAG ATGACTTCCTGCATTTGGGTGGTTCTTTTGTCACTAATTTGC CTTTTCTAAAATTGTCCTGGTTTCTTCTGCCAATTTCCCTTCT TTCTCCCCAGCATATAAAGTCTCCATCTCTGGAACCACAGTA ATATTGACATGCCCTCAGTAT |
| 20 | | P2A | GCCACCAACTTCTCTCTTTTGAAGCAGGCCGGAGATGTGGA AGAAAATCCTGGGCCC |
| 21 | | CD3 epsilon signal peptide | ATGCAATCAGGCACTCACTGGCGAGTGCTGGGTCTGTGCTT GCTCAGCGTAGGAGTATGGGGTCAA |
| 22 (similar to SEQ ID NO 12) | | Myc tag | GAG CAGAAG CTCA TCAGTGAAGAAGA TCTG |
| 23 (similar to SEQ ID NO 13) | | hu anti-HLA-A2 VH (clone: 3PF12) | CAAGTGCAACTGGTGCAGTCAGGAGGAGGAGTTGTGCAGC CAGGAGGAAGTCTGCGGGTCAGTTGCGCCGCGTCAGGCGT TACTCTCTCCGATTATGGAATGCATTGGGTCAGACAGGCAC CAGGCAAAGGTCTGGAATGGATGGCATTCATTCGCAATGAC GGGTCGGATAAGTATTACGCTGACAGCGTAAAGGGCAGGTT TACAATCTCTCGGGATAATAGCAAAAAAACTGTCAGCCTGCA GATGAGTTCACTGCGCGCCGAGGACACCGCTGTGTACTATT GTGCTAAAAACGGCGAGTCTGGGCCATTGGATTATTGGTAC TTTGATCTCTGGGGGCGGGGAACACTGGTGACTGTGTCT |
| 24 (similar to SEQ ID 14) | | optimized (G4S)3 linker | GGAGGTGGTGGATCAGGAGGTGGAGGTTCCGGAGGTGGAG GAAGT |
| 25 8simlila r to SEQ ID NO 15) | | hu anti-HLA-A2 VL (clone: 3PF12) | GACGTCGTGATGACTCAGTCTCCCTCCTCTCTGAGTGCCAG CGTTGGGGATCGCGTTACGATCACCTGCCAAGCCAGCCAG GATATCAGCAACTACTTAAATTGGTATCAGCAAAAGCCTGGA AAGGCTCCAAAGCTGCTAATCTACGACGCGTCCAACCTGGA GACCGGTGTTCCATCACGCTTCTCTGGATCTGGGTCTGGTA CCGATTTTACATTCACCATATCTTCTTTGCAACCCGAGGACT TCGCTACCTATTATTGCCAACAGTATAGCAGCTTCCCTTTAA CTTTCGGGGGAGGCACTAAAGTGGATATCAAGCGG |
| 26 (similar to SEQ ID NO 14) | | optimized (G4S)3 linker | GGAGGTGGTGGATCAGGAGGTGGAGGTTCCGGAGGTGGAG GAAGT |
| 27 | | CD3 epsilon - 1-30aa | GACGGTAACGAAGAGATGGGGGGAATCACCCAGACACCATA CAAAGTCAGCATCAGCGGCACAACGGTCATCCTGACGTGTC CTCAATAC |
| 28 | | Right homology arm | CCTGGATCTGAAATACTATGGCAACACAATGATAAAAACATA GGCGGTGATGAGGATGATAAAAACATAGGCAGTGATGAGGA TCACCTGTCACTGAAGGAATTTTCAGAATTGGAGCAAAGTGG TTATTATGTCTGCTACCCCAGAGGAAGCAAACCAGAAGATGC GAACTTTTATCTCTACCTGAGGGCAAGAGGTAATCCAGGTCT CCAGAACAGGTACCACCGGCTCTTTAGGGAGGACCATTCAA AAGGGCATTCTCAGTGATTTTCCCTAACCCAGCTCACAGTGC CCAGGCGTCTTTGCGCTTCCTCCCACACTCAATCCTGGGAC TCTCTGGTACCACACGGCATCAGTGTTTTCTGGAATATAGAT TAAACACCAATATGAGGCTTCTGGG |

**Table 3: nucleic acid sequence for HDR donor templates for TRAC (comparative sequences)**

| **SEQ ID NO** | **Homology-Directed DNA Repair (HDR) Template** | **Info HDR-Template Part** | **Sequence 5'-3'** |
|---|---|---|---|
| 19 | HDRT HLA-A2 CAR into TRAC | Full sequence | cctattaaataaaagaataagcagtattattaagtagccctgcatttcaggtttccttgagtg gcaggccaggcctggccgtgaacgttcactgaaatcatggcctcttggccaagattgata gcttgtgcctgtccctgagtcccagtccatcacgagcagctggtttctaagatgctatttccc gtataaagcatgagaccgtgacttgccagccccacagagccccgcccttgtccatcactg gcatctggactccagcctgggttggggcaaagagggaaatgagatcatgtcctaaccct |
| | | | gatcctcttgtcccacagatatccagaaccctgaccctgccgtgtaccagctgagagactc taaatccagtgacaagtctgtctgcctattcaccGCCACCAACTTCTCTCTTTT GAAGCAGGCCGGAGATGTGGAAGAAAATCCTGGGCCCATG GCTCTTCCTGTGACTGCCCTTCTGCTGCCCCTGGCTCTCCT GCTCCATGCTGCCCGGCCACAAGTGCAACTGGTGCAGTCAG GAGGAGGAGTTGTGCAGCCAGGAGGAAGTCTGCGGGTCAG TTGCGCCGCGTCAGGCGTTACTCTCTCCGATTATGGAATGC ATTGGGTCAGACAGGCACCAGGCAAAGGTCTGGAATGGATG GCATTCATTCGCAATGACGGGTCGGATAAGTATTACGCTGA CAGCGTAAAGGGCAGGTTTACAATCTCTCGGGATAATAGCA AAAAAACTGTCAGCCTGCAGATGAGTTCACTGCGCGCCGAG GACACCGCTGTGTACTATTGTGCTAAAAACGGCGAGTCTGG GCCATTGGATTATTGGTACTTTGATCTCTGGGGGCGGGGAA CACTGGTGACTGTGTCTGGAGGTGGTGGATCAGGAGGTGG AGGTTCCGGAGGTGGAGGAAGTGACGTCGTGATGACTCAGT CTCCCTCCTCTCTGAGTGCCAGCGTTGGGGATCGCGTTACG ATCACCTGCCAAGCCAGCCAGGATATCAGCAACTACTTAAAT TGGTATCAGCAAAAGCCTGGAAAGGCTCCAAAGCTGCTAAT CTACGACGCGTCCAACCTGGAGACCGGTGTTCCATCACGCT TCTCTGGATCTGGGTCTGGTACCGATTTTACATTCACCATAT CTTCTTTGCAACCCGAGGACTTCGCTACCTATTATTGCCAAC AGTATAGCAGCTTCCCTTTAACTTTCGGGGGAGGCACTAAA GTGGATATCAAGCGGGAAAGCAAGTATGGCCCCCCATGCCC TCCCTGCCCCGGACAGCCGCGGGAGCCCCAGGTCTACACC CTCCCACCCTCCAGAGATGAGCTGACCAAGAACCAGGTTTC ACTGACATGCCTGGTGAAGGGCTTCTACCCCTCTGACATTG CTGTGGAGTGGGAGAGCAATGGGCAGCCAGAGAACAACTA CAAGACCACACCTCCTGTCCTGGACAGCGACGGCTCCTTCT TCCTCTACAGTAAGCTCACTGTGGACAAGAGCCGCTGGCAG CAGGGCAATGTCTTCTCCTGCAGCGTGATGCACGAGGCCCT GCACAATGCCTATACCCAGAAGTCACTCTCTCTGAGCCCTG GAAAGAAGGATCCCAAGTTTTGGGTCCTCGTGGTGGTCGGG GGTGTGCTGGCCTGCTACAGCTTGCTGGTCACAGTGGCCTT CATCATCTTCTGGGTGCGCTCCAAGAGGAGCCGGCTGCTTC ACAGTGATTACATGAACATGACCCCCAGGAGGCCAGGACCC ACCAGGAAGCACTACCAGCCCTACGCTCCCCCGCGGGACTT TGCTGCTTACCGCAGCAGGGTCAAATTTTCTAGATCTGCAGA TGCGCCGGCCTATCAAcaaggccagaaccagctcTATAACGAGCTC AATCTAGGACGAAGAGAGGAGTACGATGTTTTGGACAAGAG ACGTGGCCGGGACCCTGAGATGGGGGGAAAGCCGAGAAGG AAGAACCCTCAGGAAGGCCTGTACAATGAACTGCAGAAAGA TAAGATGGCGGAGGCCTACAGTGAGATTGGGATGAAAGGC GAGCGCCGGAGGGGCAAGGGGCACGATGGCCTTTACCAGG GTCTCAGTACAGCCACCAAGGACACCTACGACGCCCTTCAC ATGCAGGCCCTGCCCCCTCGCTAAtaagaattctaactagagctcgctg atcagcctcgactgtgccttctagttgccagccatctgttgtttgcccctcccccgtgccttcct tgaccctggaaggtgccactcccactgtcctttcctaataaaatgaggaaattgcatcgcat tgtctgagtaggtgtcattctattctggggggtggggtggggcaggacagcaagggggag gattgggaagagaatagcaggcatgctggggagcggccgcgttaaattttgattctcaaa caaatgtgtcacaaagtaaggattctgatgtgtatatcacagacaaaactgtgctagacat gaggtctatggacttcaagagcaacagtgctgtggcctggagcaacaaatctgactttgc atgtgcaaacgccttcaacaacagcattattccagaagacaccttcttccccagcccagg taagggcagctttggtgccttcgcaggctgtttccttgcttcaggaatggccaggttctgccc agagctctggtcaatgatgtctaaaactcctctgattggtggtctcggccttatccattgccac caaaaccctctttttactaagaaacagtgagccttgttctggcagtccagagaatgacacg ggaaaaaagcagatg |
| 30 | | Left homology arm | cctattaaataaaagaataagcagtattattaagtagccctgcatttcaggtttccttgagtg gcaggccaggcctggccgtgaacgttcactgaaatcatggcctcttggccaagattgata gcttgtgcctgtccctgagtcccagtccatcacgagcagctggtttctaagatgctatttccc gtataaagcatgagaccgtgacttgccagccccacagagccccgcccttgtccatcactg gcatctggactccagcctgggttggggcaaagagggaaatgagatcatgtcctaaccct |
| | | | gatcctcttgtcccacagatatccagaaccctgaccctgccgtgtaccagctgagagactc taaatccagtgacaagtctgtctgcctattcacc |
| 31 (similar to SEQ ID NO 20) | | P2A | GCCACCAACTTCTCTCTTTTGAAGCAGGCCGGAGATGTGGA AGAAAATCCTGGGCCC |
| 32 | | Signal peptide | ATGGCTCTTCCTGTGACTGCCCTTCTGCTGCCCCTGGCTCT CCTGCTCCATGCTGCCCGGCCA |
| 33 (similar to SEQ ID NO 13) | | hu anti-HLA-A2 VH (clone: 3PF12) | CAAGTGCAACTGGTGCAGTCAGGAGGAGGAGTTGTGCAGC CAGGAGGAAGTCTGCGGGTCAGTTGCGCCGCGTCAGGCGT TACTCTCTCCGATTATGGAATGCATTGGGTCAGACAGGCAC CAGGCAAAGGTCTGGAATGGATGGCATTCATTCGCAATGAC GGGTCGGATAAGTATTACGCTGACAGCGTAAAGGGCAGGTT TACAATCTCTCGGGATAATAGCAAAAAAACTGTCAGCCTGCA GATGAGTTCACTGCGCGCCGAGGACACCGCTGTGTACTATT GTGCTAAAAACGGCGAGTCTGGGCCATTGGATTATTGGTAC TTTGATCTCTGGGGGCGGGGAACACTGGTGACTGTGTCT |
| 34 (similar to SEQ ID NO 14) | | optimized (G4S)3 linker | GGAGGTGGTGGATCAGGAGGTGGAGGTTCCGGAGGTGGAG GAAGT |
| 35 (similar to SEQ ID NO 15) | | hu anti-HLA-A2 VL (clone: 3PF12) | GACGTCGTGATGACTCAGTCTCCCTCCTCTCTGAGTGCCAG CGTTGGGGATCGCGTTACGATCACCTGCCAAGCCAGCCAG GATATCAGCAACTACTTAAATTGGTATCAGCAAAAGCCTGGA AAGGCTCCAAAGCTGCTAATCTACGACGCGTCCAACCTGGA GACCGGTGTTCCATCACGCTTCTCTGGATCTGGGTCTGGTA CCGATTTTACATTCACCATATCTTCTTTGCAACCCGAGGACT TCGCTACCTATTATTGCCAACAGTATAGCAGCTTCCCTTTAA CTTTCGGGGGAGGCACTAAAGTGGATATCAAGCGG |
| 36 | | IgG4 hinge | GAAAGCAAGTATGGCCCCCCATGCCCTCCCTGCCCC |
| 37 | | IgG1 CH3 | GGACAGCCGCGGGAGCCCCAGGTCTACACCCTCCCACCCT CCAGAGATGAGCTGACCAAGAACCAGGTTTCACTGACATGC CTGGTGAAGGGCTTCTACCCCTCTGACATTGCTGTGGAGTG GGAGAGCAATGGGCAGCCAGAGAACAACTACAAGACCACAC CTCCTGTCCTGGACAGCGACGGCTCCTTCTTCCTCTACAGT AAGCTCACTGTGGACAAGAGCCGCTGGCAGCAGGGCAATG TCTTCTCCTGCAGCGTGATGCACGAGGCCCTGCACAATGCC TATACCCAGAAGTCACTCTCTCTGAGCCCTGGAAAG |
| 38 | | CD28 transmembran e | AAGGATCCCAAGTTTTGGGTCCTCGTGGTGGTCGGGGGTGT GCTGGCCTGCTACAGCTTGCTGGTCACAGTGGCCTTCATCA TCTTCTGGGTG |
| 39 | | CD28 endodomain | CGCTCCAAGAGGAGCCGGCTGCTTCACAGTGATTACATGAA CATGACCCCCAGGAGGCCAGGACCCACCAGGAAGCACTAC CAGCCCTACGCTCCCCCGCGGGACTTTGCTGCTTACCGCAG C |
| 40 | | CD3 zeta endodomain | AGGGTCAAATTTTCTAGATCTGCAGATGCGCCGGCCTATCAA caaggccagaaccagctcTATAACGAGCTCAATCTAGGACGAAGAG AGGAGTACGATGTTTTGGACAAGAGACGTGGCCGGGACCCT GAGATGGGGGGAAAGCCGAGAAGGAAGAACCCTCAGGAAG GCCTGTACAATGAACTGCAGAAAGATAAGATGGCGGAGGCC TACAGTGAGATTGGGATGAAAGGCGAGCGCCGGAGGGGCA AGGGGCACGATGGCCTTTACCAGGGTCTCAGTACAGCCACC AAGGACACCTACGACGCCCTTCACATGCAGGCCCTGCCCCC TCGCTAA |
| 41 | | bgH terminator sequence | taagaattctaactagagctcgctgatcagcctcgactgtgccttctagttgccagccatctg ttgtttgcccctcccccgtgccttccttgaccctggaaggtgccactcccactgtcctttcctaa taaaatgaggaaattgcatcgcattgtctgagtaggtgtcattctattctggggggtggggtg gggcaggacagcaagggggaggattgggaagagaatagcaggcatgctggggagc ggccgcgttaa |
| 42 | | Right homology arm | attttgattctcaaacaaatgtgtcacaaagtaaggattctgatgtgtatatcacagacaaa actgtgctagacatgaggtctatggacttcaagagcaacagtgctgtggcctggagcaac aaatctgactttgcatgtgcaaacgccttcaacaacagcattattccagaagacaccttctt ccccagcccaggtaagggcagctttggtgccttcgcaggctgtttccttgcttcaggaatgg ccaggttctgcccagagctctggtcaatgatgtctaaaactcctctgattggtggtctcggcc ttatccattgccaccaaaaccctctttttactaagaaacagtgagccttgttctggcagtcca gagaatgacacgggaaaaaagcagatg |

**Table 4: primers for donor template amplification (inventive sequences)**

| Any of the sequences SEQ ID 43 to 46 provided in the table below, any combination of sequences, or any marked sub-regions of the sequence may represent an embodiment of the invention and/or be employed in any of the aspects or embodiments of the invention described herein. | | | |
|---|---|---|---|
| **SEQ ID NO** | **Sequence 5'-3'** | **name** | **comment** |
| 43 | TCTTCCCAGCATTGCATTCTCAACT | P557_CD3e-ex6-amp-HA-F | Primer for donor template amplification |
| 44 | CCCAGAAGCCTCATATTGGTGTTTAATCT | P558_CD3e-ex6-amp-HA-R | Primer for donor template amplification |
| 45 | TACCCAACCAAGAGCGTGTTTTTCT | P559_CD3e-ex3-amp-HA-F | Primer for donor template amplification |
| 46 | ATTCAGACATGTGATTCTGAACACACATCC | P560_CD3e-ex3-amp-HA-R | Primer for donor template amplification |
| 57 | cctattaaataaaagaataagcagtattattaagtagccctgc | P75_ourTRAC- amp-HA-F | Primer for donor template amplification |
| 58 | catctg cttttttcccgtgtcattct | P76_ourTRAC- amp-HA-R | Primer for donor template amplification |

**Table 5: Amino acid sequenced of antigen binding domains (inventive sequences)**

| Any of the sequences SEQ ID 47 to 56 provided in the table below, any combination of sequences, or any marked sub-regions of the sequence may represent an embodiment of the invention and/or be employed in any of the aspects or embodiments of the invention described herein. | | | |
|---|---|---|---|
| **SEQ ID NO** | **Homology-Directed DNA Repair (HDR) Template Part** | **Info scFV Part** | **Amino acid sequence** |
| 47 | hu anti-HLA-A2 scFV fragment (clone: 3PF12) | Full sequence | QVQLVQSGGGVVQPGGSLRVSCAASGVTLSDYGMHWVRQAP GKGLEWMAFIRNDGSDKYYADSVKGRFTISRDNSKKTVSLQM SSLRAEDTAVYYCAKNGESGPLDYWYFDLWGRGTLVTVSGGG GSGGGGSGGGGSDVVMTQSPSSLSASVGDRVTITCQASQDIS NYLNWYQQKPGKAPKLLIYDASNLETGVPSRFSGSGSGTDFTF TISSLQPEDFATYYCQQYSSFPLTFGGGTKVD |
| 48 | | hu anti-HLA-A2 VH (clone: 3PF12) | QVQLVQSGGGVVQPGGSLRVSCAASGVTLSDYGMHWVRQAP GKGLEWMAFIRNDGSDKYYADSVKGRFTISRDNSKKTVSLQM SSLRAEDTAVYYCAKNGESGPLDYWYFDLWGRGTLVTVS |
| 49 | | optimized (G4S)3 linker | GGGGSGGGGSGGGGS |
| 50 | | hu anti-HLA-A2 VL (clone: 3PF12) | DVVMTQSPSSLSASVGDRVTITCQASQDISNYLNWYQQKPGK APKLLIYDASNLETGVPSRFSGSGSGTDFTFTISSLQPEDFATY YCQQYSSFPLTFGGGTKVD |
| 51 | hu anti-HLA-A2 VH (clone: 3PF12) | H-CDR1 | GVTLSDY |
| 52 | | H-CDR2 | RNDGSD |
| 53 | | H-CDR3 | NGESGPLDYWYFDL |
| 54 | hu anti-HLA-A2 VL (clone: 3PF12) | L-CDR1 | QASQDISNYLN |
| 55 | | L-CDR2 | DASNLET |
| 56 | | L-CDR3 | QQYSSFPLT |

### FIGURES

The invention is further described by the following figures. These are not intended to limit the scope of the invention but represent preferred embodiments of aspects of the invention provided for greater illustration.

### Brief Description of the Figures

**Figure 1****:** TCR replaced A2 CAR Treg function in vitro but suffer from poor expansion.
**Figure 2****:** Identification of a CD3 epsilon gene editing strategy to create TruC+ T cells after integration of scFv cDNA
**Figure 3****:** Repetitive stimulation leads to preferential expansion of CD3e-TruC+ Treg over unedited Tregs or TRAC-replaced CAR TregA. Schematic pipeline of CD3e-TruC+ and TRAC-CAR Treg cell generation and expansion.
**Figure 4****:** Gene edited and ex vivo expanded Treg retain their canonical phenotype and epigentic identity
**Figure 5****:** Activation profiles of CD3e-TruC Treg cells compared to TRAC-CAR and WT Treg cells upon antigen HLA-A2 specific stimulation.
**Figure 6****:** A2 CD3e-TruC+ Tregs functionally suppress autologous Tconv proliferation in vitro regardless of polyclonal or HLA-A2 antigen-specific stimulation.
**Figure 7****:** Purity check of polyclonal Treg sorting
**Figure 8****:** CD3e-TruC Treg specifically recognize HLA-A2 antigen
**Figure 9****:** CD3e-TruC Tregs rapidly upregulated TCR downstream molecular phosphorylation upon antigen HLA-A2 and αCD3 stimulation.
**Figure 10****:** Purity check of gene edited Treg sorting.
**Figure 11****:** Histogram plots of autologous Tconv proliferation without Treg.
**Figure 12****:** Alternative donor also showed that A2 CD3e-TruC+ Tregs functionally suppress autologous Tconv proliferation in vitro regardless of polyclonal or HLA-A2 antigen-specific stimulation.

### Detailed Description of the Figures

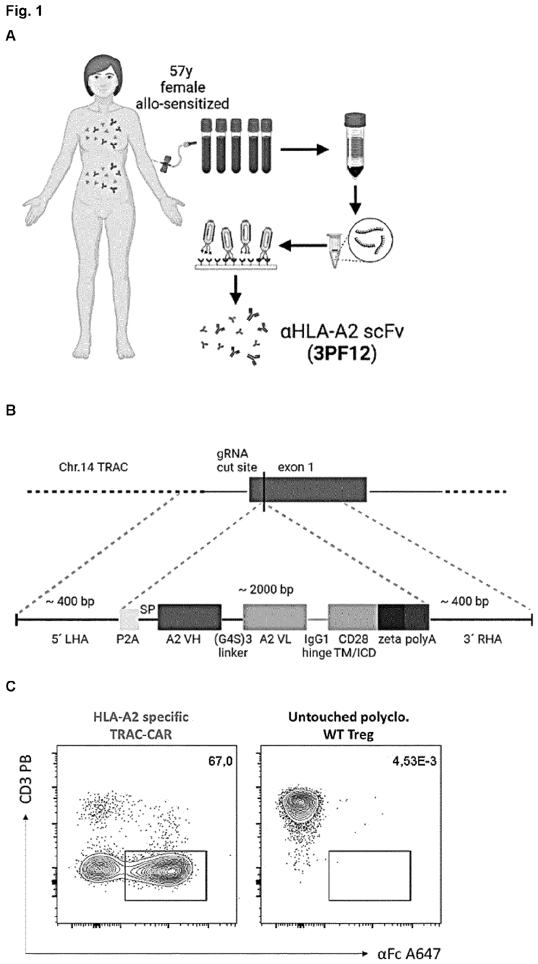

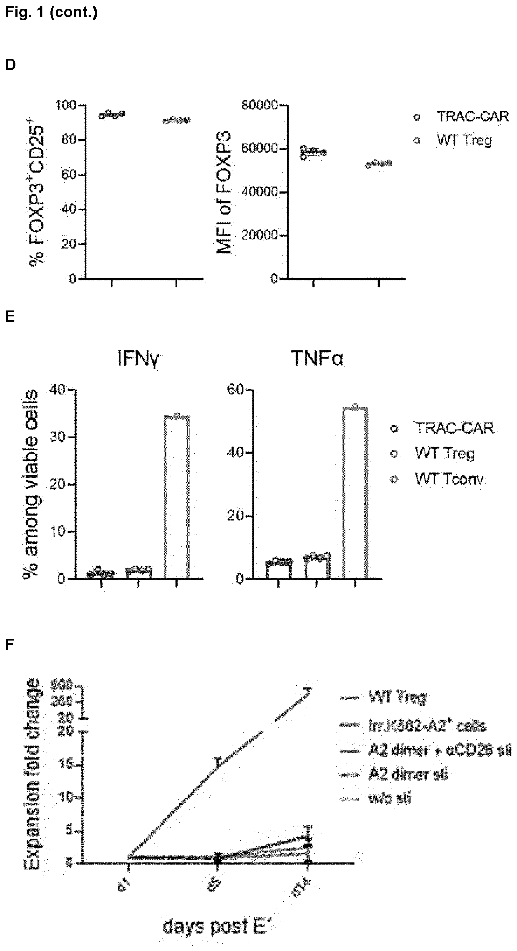

**Figure 1****:** (A) Anti-human HLA-A2 scFv clone 3PF12 with medium affinity was used in this study, which is based on Watkins et al. (2000) . (B) HDR-mediated integration of a donor template into TRAC locus. Donor template contains 400 bp left homology arm (LHA), self-cleaving peptide P2A, signal peptide (SP), heavy chain and light chain of HLA-A2 scFv with 3 × G4S linker in between, IgG1 hinge (used for detecting CAR integration), CD28 transmembrane domain (TD) and intracellular domain (ICD), zeta chain, followed by a poly (A) tail and 400 bp right homology arm (RHA). (C) Dot plots of αFc A647 vs. CD3 PB stained on HLA-A2 specific TRAC-CAR Treg and untouched wild type (WT) Treg cells. Gated on live single lymphocytes. (D) Percentages of FOXP3 and CD25 double positive cells (left) and FOXP3 MFI (right) between TRAC-CAR and WT Treg cells. (E) Comparison of percentage inhibition between suppression assays performed with TRAC-CAR and WT Treg cells. (F) Expansion fold change during 2 week expansion post electroporation of TRAC-CAR Treg cells upon either irradiated K562.A2+ cells (ratio 1:1), or plate-coated 5 µg/ml A2 dimer with or without 1 µg/ml αCD28, or without stimulation. WT Treg cells with Treg expansion bead stimulation (ratio 1:1) as control. Treg cells were stimulated every 2 to 3 days. N=3

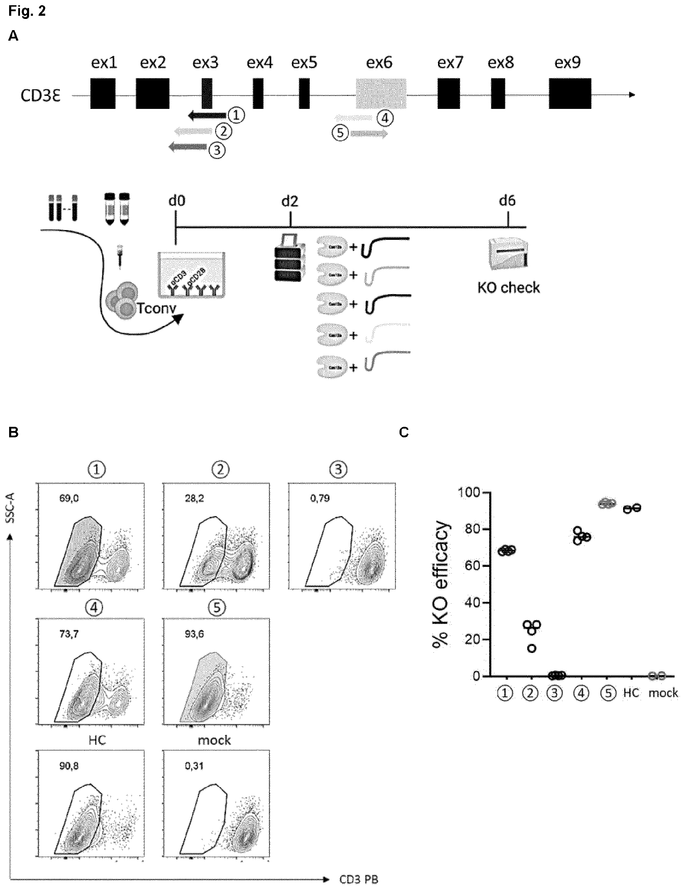

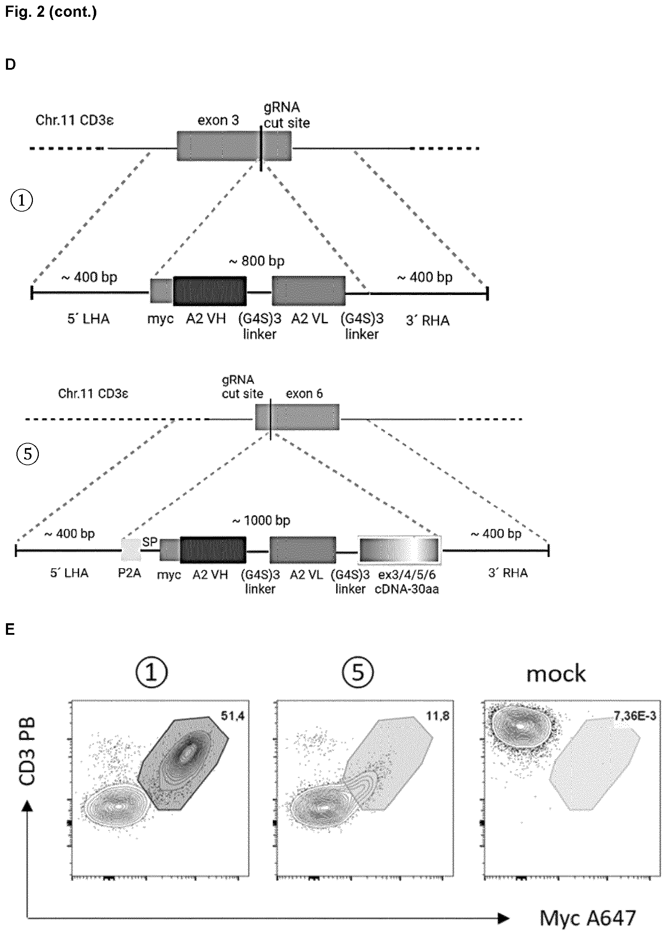

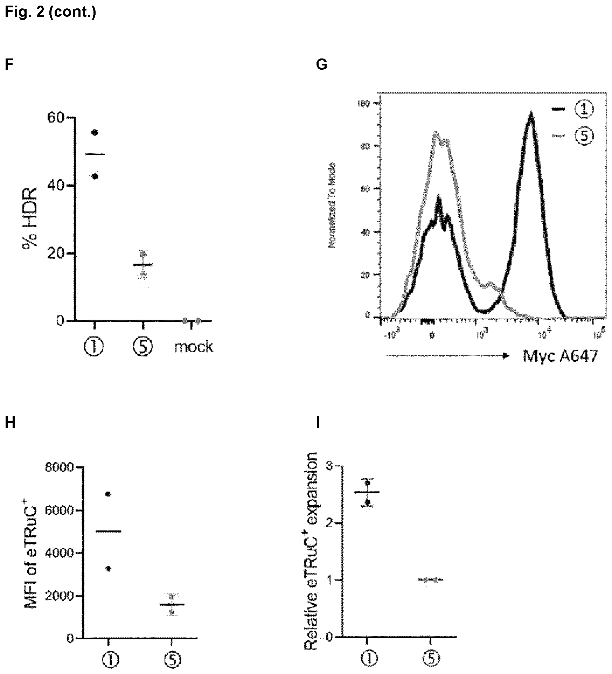

**Figure 2****:** (A) Five gRNA specifically targeting on exon 3 and exon 6 of CD3 epsilon locus were identified and knock out (KO) experiments were performed in human conventional T cells. (B) Representative dot plots of CD3 PB against SSC-A, KO cells were CD3 negative (left) (C) Summary of KO efficacy of each gRNA, n= 2 or 4 (right). (D) Donor template design and integration into gRNA1 cut site (left) and gRNA5 cut site (right). (E) Representative dot plots of myc A647 against CD3 PB, successfully gene edited cells were CD3 and myc double positive (F) HDR efficiency comparison for donor templates integrated in gRNA1 and gRNA5 cut sites. (G) Histogram overlay of myc+ cells integrated in gRNA1 and gRNA5 cut sites. (H) MFI of CD3e-TruC+ cells integrated in gRNA1 and gRNA5 cut sites. (I) Relative CD3e-TruC+ cell expansion upon 4 days after electroporation that were integrated in gRNA1 and gRNA5 cut sites (normalized to gRNA5).

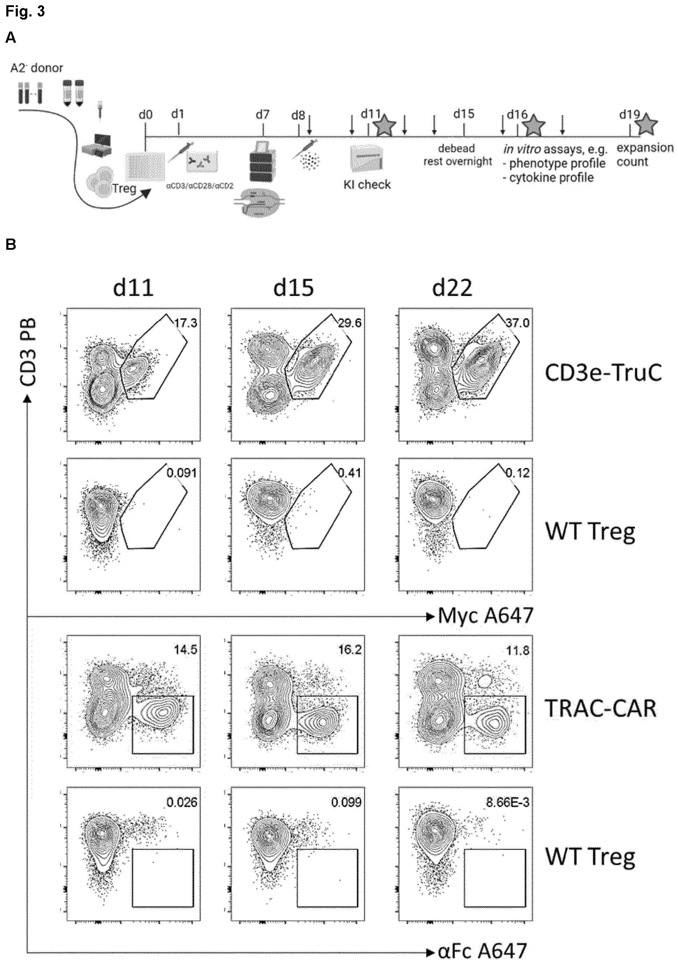

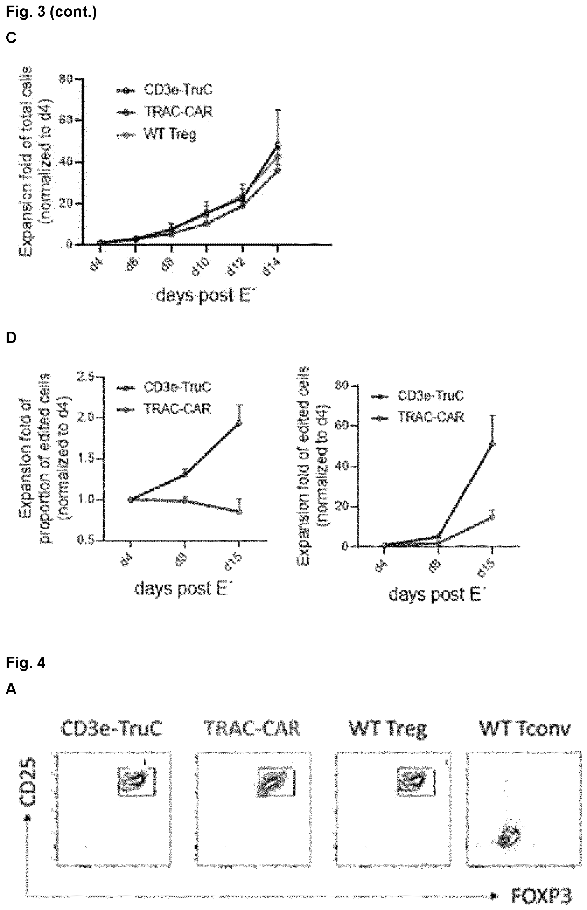

**Figure 3****:** (A) Schematic pipeline of CD3e-TruC+ and TRAC-CAR Treg cell generation and expansion. (B) Representative dot plots of CD3e-TruC+ indicated by myc and CD3 double postive or HLA-A2 and myc double positive (left) and representative dot plots of TRAC-CAR indicated by αFc positive but CD3 negative. (C) Expansion fold change of total viable cells among CD3e-TruC, TRAC-CAR compared with WT Treg cells. N=4 (D) Expansion fold change of gene edited cells between CD3e-TruC and TRAC-CAR Treg cells. N=4.

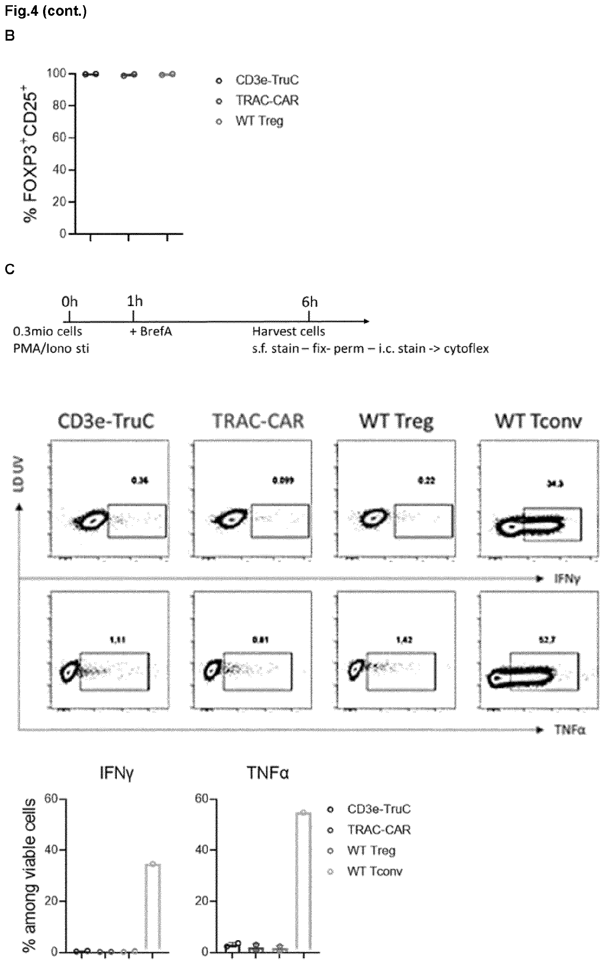

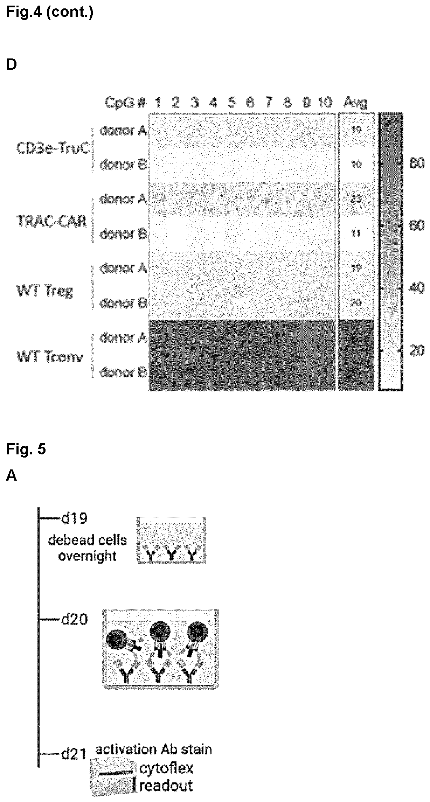

**Figure 4****:** (A) Representative dot plots of FOXP3 against CD25 among CD3e-TruC, TRAC-CAR, WT Treg and WT Tconv cells. (B) Percentages of FOXP3 and CD25 double positive cells (left) and FOXP3 MFI (right) of CD3e-TruC compared to TRAC-CAR and WT Treg cells. n=4 (C) Cytokine IFN-γ and TNF-α expression level of CD3e-TruC, TRAC-CAR and WT Treg upon PMA and Inonmycin stimulation. WT Tconv used as high control. Schematic pipeline of CD3e-TruC+ and TRAC-CAR Treg cell generation and expansion. (D) Treg specific methylation region analysis demonstrates low methylation on all 10 CpGs in unedited Treg (WT), CD3-TruC and TRAC-replaced CAR Treg, but not conventional T cells (WT Tconv).

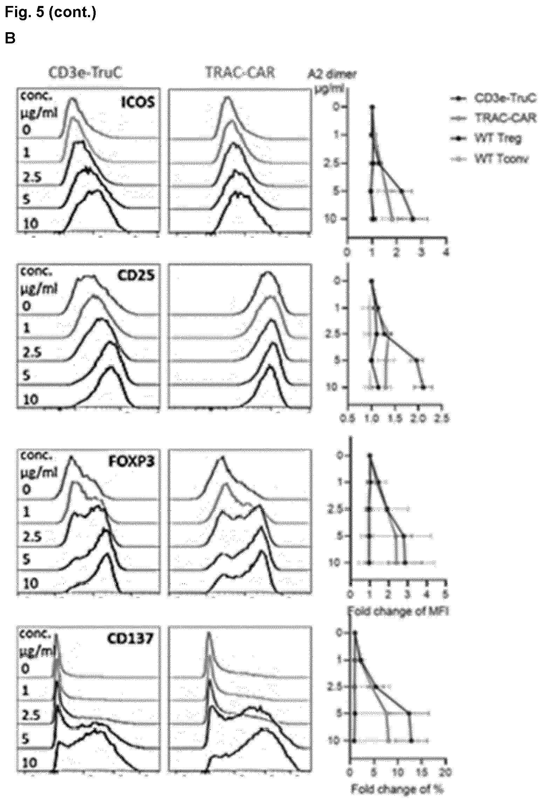

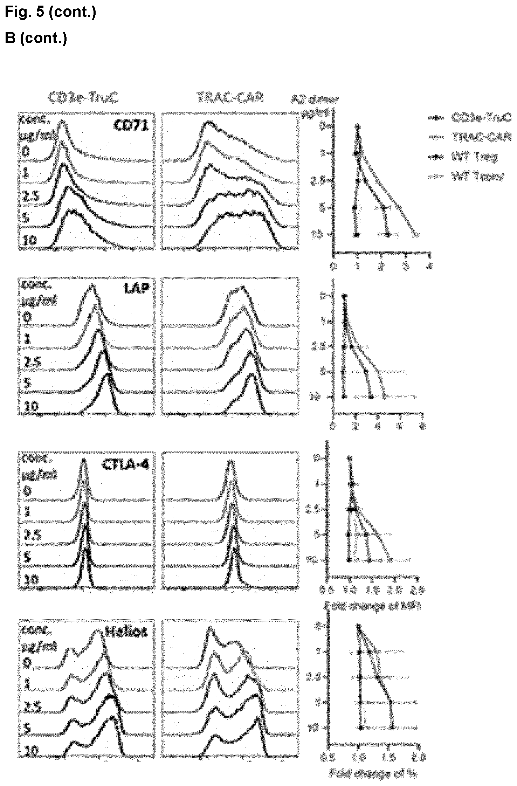

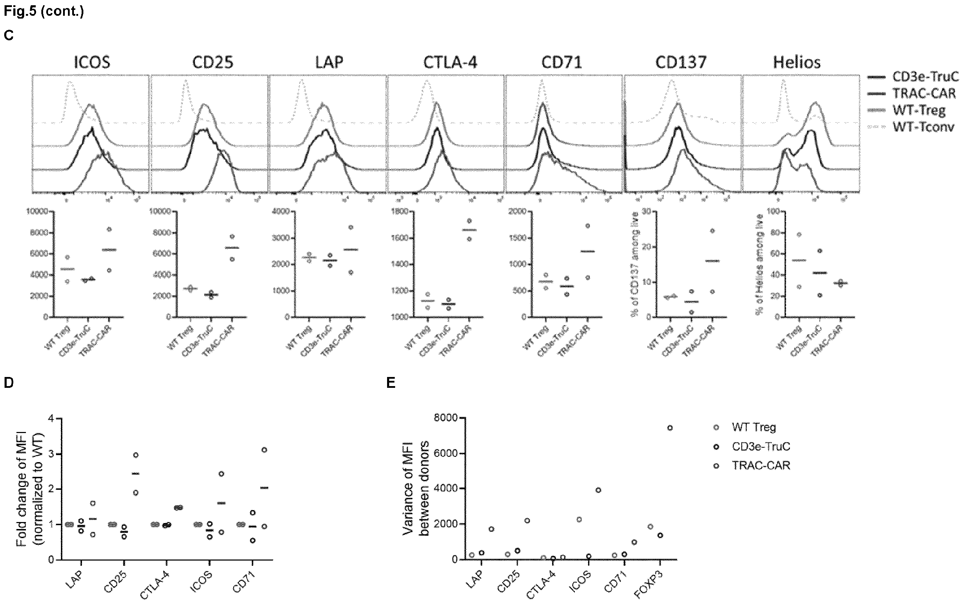

**Figure 5****:** (A) Experimental setup of activation assay. (B) Representative offset histograms and summary of each activation marker of CD3e-TruC and TRAC_CAR upon a gradient concentration of plate coated HLA-A2 stimulation. (C) Representative offset histograms (upper) and summary (lower) of each activation marker of CD3e-TruC and TRAC_CAR compared to WT Treg cells, WT Tconv (dotted line) as negative control. N=2 (D) Fold change of MFI of activation markers of CD3e-TruC and TRAC_CAR, normalized to WT Treg cells. (E) Variance of activation marker MFI between two donors, by comparing CD3e-TruC, TRAC_CAR and WT Treg cells.

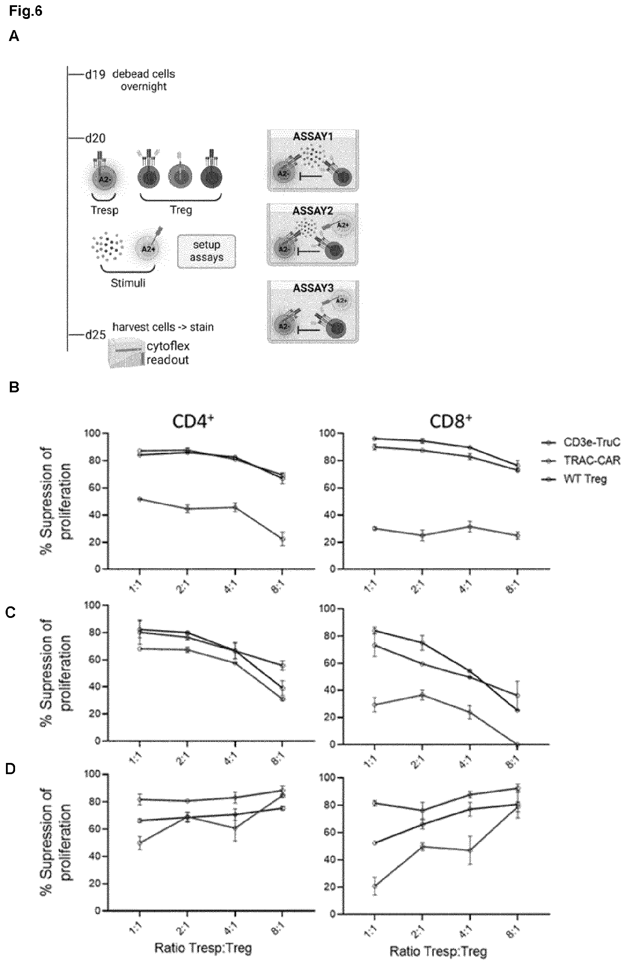

**Figure 6****:** (A) Experimental setup of suppression assays. Comparison of percentage inhibition for CD4+ Tconv (left) and CD8+Tconv (right) between suppression assays performed with CD3e-TruC, TRAC-CAR and WT Treg cells upon polyclonal bead alone stimulation (B), upon polyclonal bead and CD3 depleted HLA-A2+ cell stimulation (C) or upon CD3 depleted HLA-A2+ cell alone stimulation (D).

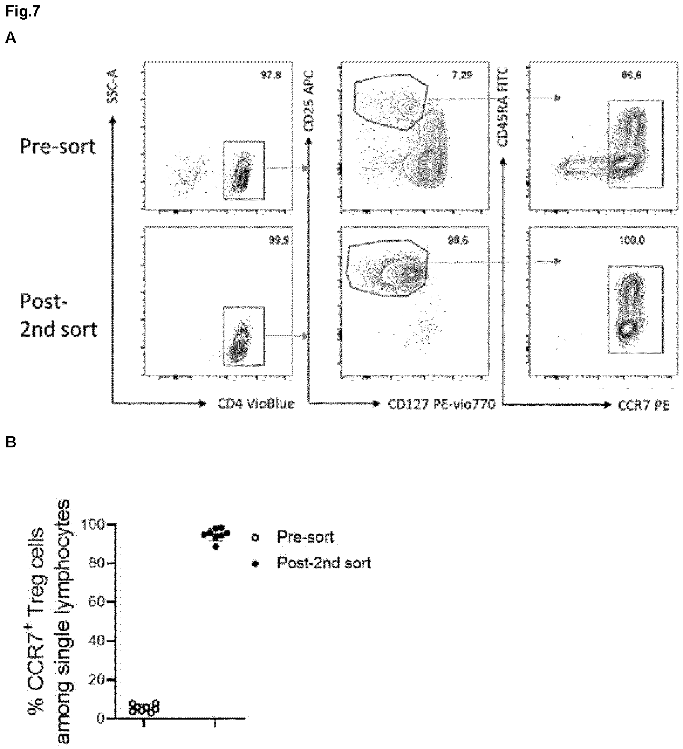

**Figure 7****:** (A) Representative dot plots of CD4+CD25highCD127-CCR7+ Treg cells pre-sort (upper) and post 2 rounds of sort by Tyto. Gated on single viable lymphocytes; highly purified polyclonal Treg cells used for HLA-A2 specific CD3e-TruC and TRAC-CAR Treg cells. (B) Percentages of CCR7+ Treg cells among single lymphocytes before and after sorting with Tyto sorter. n=8.

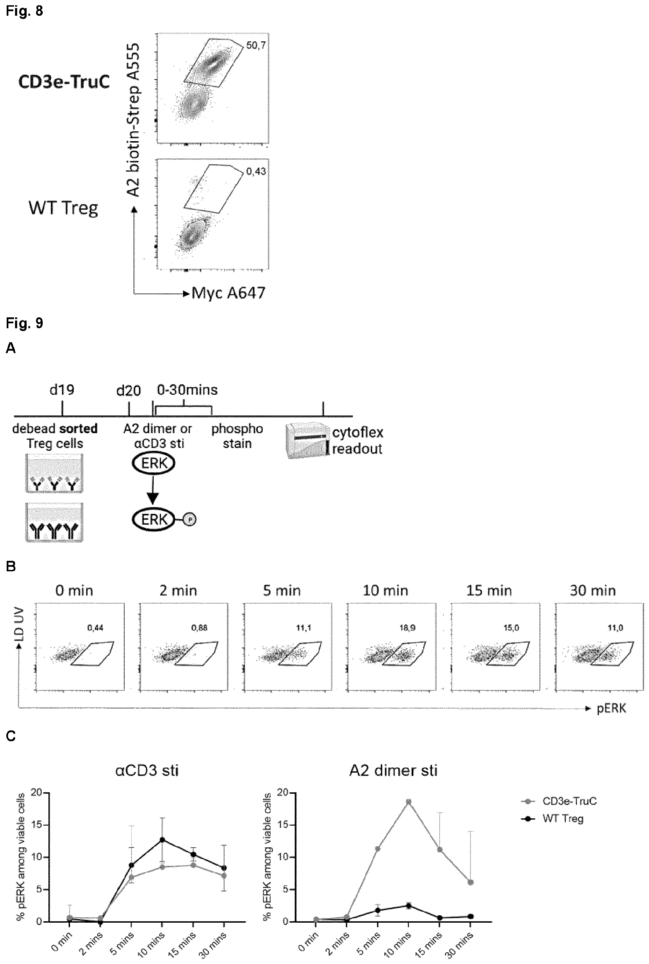

**Figure 8****:** Representative dot plots of CD3e-TruC Treg (upper) and WT Treg (lower) showing that CD3e-TruC Treg specifically recognize HLA-A2 antigen.

**Figure 9****:** (A) Experimental setup of phosphorylation assay. (B) Representative FACS plots showing proportion of phosphorylated ERK among viable cells upon 5 µg/ml HLA-A2 stimulation. (C) Kinetics of phosphorylated ERK among viable cells upon either 10 µg/ml αCD3 (left) or 5 µg/ml HLA-A2 (right) stimulation.

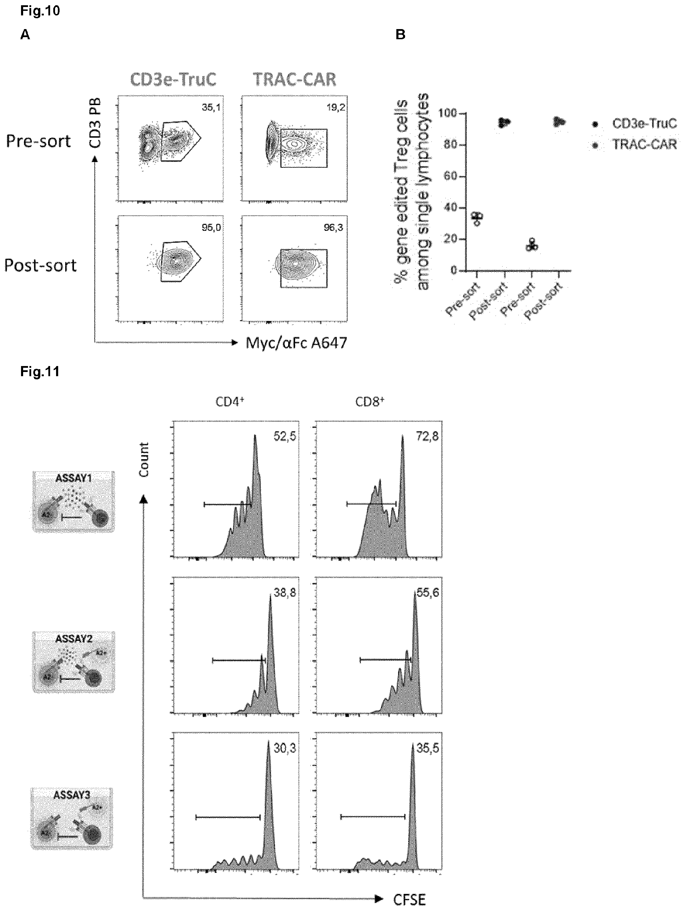

**Figure 10****:** (A) Representative dot plots of CD3+Myc+ CD3e-TruC Treg (left) and CD3-aFc+ TRAC-CAR Treg (right) cells pre-sort (upper) and post-sort by Aria II. Gated on single viable CD4+ T lymphocytes. (B) Percentage of gene edited Treg cells among single lymphocytes displayed for CD3e-TruC cells and TRAC-CAR cells.

**Figure 11****:** Histogram plots of autologous Tconv proliferation without Treg. CD4+ (left) and CD8+ (right) Tconv cells were co-culture with either aCD3/CD28 bead at a ratio of 1:1 (upper) or bead together with HLA-A2+ CD3 depleted PBMCs at a ratio of 1:1:1 (middle) or HLA-A2+ CD3 depleted PBMCs alone at a ratio of 1:1 (lower).

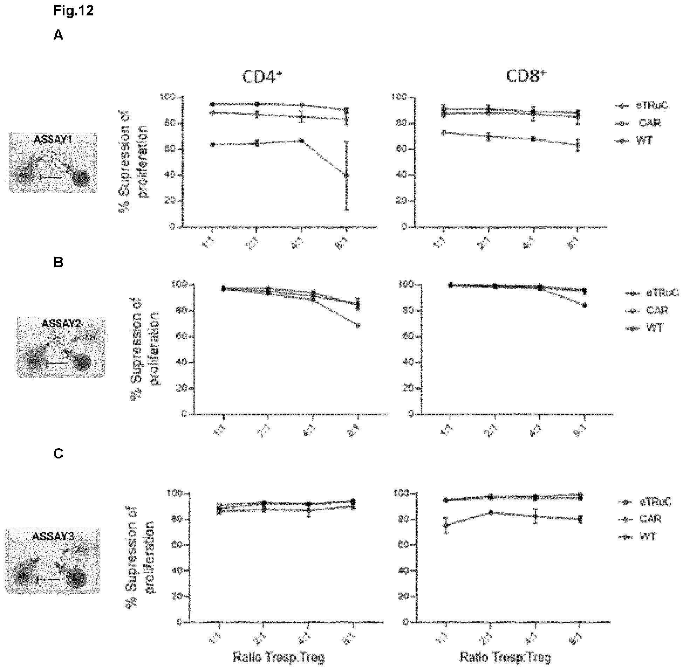

**Figure 12****:** Comparison of percentage inhibition for CD4+ Tconv (left) and CD8+Tconv (right) between suppression assays performed with CD3e-TruC, TRAC-CAR and WT Treg cells upon polyclonal bead alone stimulation (A), upon polyclonal bead and CD3 depleted HLA-A2+ cell stimulation (B) or upon CD3 depleted HLA-A2+ cell alone stimulation (C).

### EXAMPLES

The invention is demonstrated through the examples disclosed herein. The examples provided represent embodiments of the invention and are not intended to limit the scope of the invention. The examples are to be considered as providing a non-limiting illustration and technical support for carrying out the invention.

The nucleic acid constructs employed in the examples and comparative examples are disclosed in the sequence tables above (Table 1 to Table 4).

### Comparative example 1: TCR replaced A2 CAR Treg (TRAC-CAR Treg) were successfully generated via non-viral gene editing but suffered from poor expansion.

To generate an HLA-A2-specific CAR construct for Treg engineering, the antigen-binding domain of 2nd generation CAR was replaced with an HLA-A2-specific scFv which was originally derived from an allo-sensitized 57-year-old female patient (Fig. 1 A). The CAR was cloned into a homology-directed repair template (HDRT) for insertion into the TRAC locus as previously described (Kath et al., 2022) (Fig. 1 B). Following PBMC isolation from peripheral blood of HLA-A2-negative healthy donors, Treg were enriched for CD4+, CD25high, CD127low and CCR7+ (Fig. 7). Then, Treg were activated using polyclonal stimulation and expanded in the presence of high dose IL-2 and the mTOR inhibitor rapamycin. After 7-9 days expansion, the proliferating Treg were harvested and electroporated with linear double stranded (ds)DNA HDRT and a pre-complexed RNP containing TRAC-specific guide RNA (gRNA) and recombinant *Streptococcus pyogenes* (S.p.) Cas9 protein. The resulting TRAC-CAR+ Treg were predominantly CD3-negative (Fig. 1 C), retained canonical markers of Treg identity - expressed high levels of CD25 and FOXP3 (Fig. 1 D) and secreted minimal amounts of proinflammatory cytokines, e.g. IFN-γ and TNF-α (Fig. 1 E). However, TRAC-CAR Treg could not be efficiently expanded via CAR stimulation (Fig. 1 F): Re-stimulation on plate-bound recombinant HLA-A2-IgG-dimer protein every 2-3 days with and without anti-CD28 mAb yielded expansion rates between 1.60 and 2.57 respectively. Addition of irradiated HLA-A2 overexpressing K562 cells improved expansion from 0.5-fold (no stimulation) up to 5-fold. In contrast, polyclonally expanded CD4 Treg could expand up to 500-fold within a similar expansion period (Fig. 1 F). In summary, the suboptimal expansion of TRAC-CAR Treg is prohibitive to create a stable protocol at clinical scale.

### Example 1: Identification of a CD3 epsilon gene editing strategy to create TruC+ T cells after integration of scFv cDNA

Homology-directed repair with programmable nucleases depends on efficient installation of DNA double strand breaks. Thus, first 5 potential gRNA candidates for the CRISPR-Cas12a nuclease from *Acidaminococcus* species (A.s.Cas12a) with no / minimal predicted off-targets for their ability to disrupt CD3e in exon 3 or 6 were screened (Fig. 2 A). Synthetic gRNAs and recombinant A.s.Cas12 Ultra (Zhang et al., 2021) were co-electroporated in polyclonally activated T cells, and CD3e expression was measured by flow cytometry as a readout. Four days after nucleofection, T cells treated with AsCas12a gRNA #1 and #5 displayed the highest KO rate compared to the other gRNAs in their respective exon (Fig. 2 B, C). For each gRNA, we designed an HDRT with 400 bp flanking homology arms to insert a myc-tag for detection purposes, the HLA-A2-scFv cDNA and a linker to attach the antigen-binding domain onto the CD3 epsilon protein (Fig. 2 D). After transfection of gRNA and the respective dsDNA HDRTs, we were able to detect myc+ TruC T cells (Fig. 2 E). Knock-in rates, expression level and overall number of TruC+ T cells was in highest in cells edited in CD3e exon 3 (Fig. 2 F, G, H, I).

### Example 2: Repetitive stimulation leads to preferential expansion of CD3e-TruC+ Treg over TCR-negative Treg.

Using the non-viral gene editing protocol for Treg, HLA-A2-specific CD3e-TruC, TRAC-CAR Treg and wildtype (WT), unedited Treg were generated for subsequent functional tests (Fig. 3 A). Gene editing of CD3e was higher in conventional T cells. CD3e-TruC+ rates in Treg were comparable or higher than TRAC-CAR knock-in in the same donor (Fig. 3 B). TruC+ Treg were predominantly CD3+. Efficient engagement of TruC with HLA-A2 was further verified by staining with biotin-labeled recombinant HLA-A2 (Fig. 8). To validate signaling after TCR or antigen receptor engagement (Fig. 9 A), we performed flow cytometric analysis of phosphorylated extracellular signal-regulated kinase (ERK). Upon stimulation with plate-bound anti-CD3 mAb, CD3e-TruC displayed time-dependent phosphorylation of ERK at similar rates to WT Treg (Fig. 9 C left). Stimulation with plate-bound HLA-A2-IgG protein led to ERK phosphorylation in CD3e-TruC but not unedited Treg (WT Treg) (Fig. 9 C right).

During polyclonal expansion with anti-CD3/28 beads, CD3e-TruC Treg expanded similar to WT Treg (Fig. 3 C).TRAC-CAR Treg expanded at substantially lower rate during anti-CD3/28 bead stimulation (Fig. 3 D). Due to a preferential expansion of TruC+/CD3+ Tregs over TCR-negative Treg during expansion with repetitive anti-CD3/28 bead stimulation (Fig. 3B), the relative and absolute fold expansion of HLA-A2-specific Treg was higher within the CD3e-TruC Treg, compared to TRAC-CAR Treg (Fig. 3 D). Therefore, CD3e-TruC Treg can be expanded more efficiently than TRAC-replaced CAR T cells.

### Example 3: Gene edited and in vitro expanded Treg retained their canonical phenotypic and epigenetic identities.

After expansion of a total of 16 days, CD3e-TruC, TRAC-CAR and WT Treg all retained the typical Treg phenotype (Fig. 4 A, B). Further, untouched and gene edited Treg did not secrete Th1 cytokines, IFNγ and TNFα, after polyclonal restimulation (Fig. 4 C). Analysis of the Treg specific demethylation region (TDSR) in the Foxp3 gene locus was performed to confirm that Treg retained epigenetic identity with or without gene editing (Fig. 4D). As expected, CD3e-TruC and TRAC-replaced CAR Treg retained low TSDR methylation like untouched Treg. In contrast, unedited conventional T cells from the same region were highly methylated (Fig. 4D).

### Example 4: Activation profiles of CD3e-TruC Treg cells compared to TRAC-CAR and WT Treg cells upon antigen HLA-A2 specific stimulation.

To compare the activation of TRAC-CAR or CD3e-TruC Treg, the expression level of different Treg activation markers in response to different amount of plate-bound HLA-A2-IgG protein was monitored (Fig. 5 A). Both CD3e-TruC and TRAC-CAR Treg displayed dose-dependent upregulation of ICOS, CD25, Foxp3, CD137, CD71, LAP and to a limited extend also CTLA4 and Helios (Fig 5 B). CD3e-TruC displayed a stronger relative upregulation of ICOS, CD25, CD137 compared to TRAC-CAR Treg and their respective baseline expression without stimulation (Fig. 5 B). Of note, TRAC-CAR Treg displayed significantly higher baseline expression of CD25, CTLA-4, CD71 and CD137, in comparison to both CD3e-TruC and WT Treg and may indicate antigen-independent signaling of the CAR (Fig. 5 C). In contrast, CD3e-TruC and WT Treg share similar activation marker expression profile at baseline (Fig. 5 D). Furthermore, the TRAC-CAR Treg showed higher donor variance in the respective MFI of LAP, CD25, CTLA-4, ICOS, CD71 and FOXP3 (Fig. 5. E).

### Example 5: A2 CD3e-TruC+ Tregs functionally suppress autologous Tconv proliferation in vitro regardless of polyclonal or HLA-A2 antigen-specific stimulation.

Suppression of the unwanted proliferation of conventional T cells specific to auto- or allo-antigens is the primary purpose of Treg and this can be modelled in vitro. To compare the effect of different means of stimulation for conventional responder T cells (Tresp) and Treg, three different proliferation suppression assay setups for comparison of our Treg candidates were used (Fig. 6. A). The first assay (1) represents the standard proliferation suppression assay using autologous T cells (Tresp) stimulated with anti-CD3/28 beads together with different amounts of Tregs, but lacking a HLA-A2-specific stimulant. In this assay, WT Treg and CD3-TruC Treg significantly outperformed TRAC-CAR Treg (Fig. 6 B, Fig.12 A). In the second assay (2), additional HLA-A2+ CD3-depleted PBMCs were added to serve as stimulators for the CAR/TruC receptors. This partially restored the suppressive action of TRAC-CAR Treg. However, CD3e-TruC and WT Treg still displayed higher suppressive capacities toward CD8+ Tresp in one of two donors (Fig. 6 C, Fig. 12 B. The third assay (3) aimed to model allo-reactive T cell proliferation towards the unmatched HLA-A2+ CD3-depleted PBMCs and no additional anti-CD3/28 beads were included. As expected, the overall percentage of proliferating Tresp was much lower (Fig. 11). Surprisingly, at lower Tresp:Treg ratios, higher proliferation suppression was observed in one donor (Fig. 6 D). As we titrated the amount of CD3-depleted PBMCs from an HLA-A2+ donor to the overall cell number, we believe this may be an artifact. In one donor the CD3e-TruC Treg displayed the highest suppressive capacity at all tested Tresp:Treg ratios (Fig. 6 D). In the other donor, CD3e-TruC and TRAC-CAR Treg outperformed WT Treg in the suppression of CD8+ Tresp (Fig. 12 C). Overall, CD3e-TruC Treg showed high suppressive capacity in autologous and allogeneic proliferation suppression assays in vitro.

### Methods and Materials:

*Polyclonal Treg cell isolation:* All experiments were performed in accordance with the Declaration of Helsinki. Peripheral blood was obtained from healthy human adults after informed consent (Charité ethics committee approval EA4/091/19). HLA-A2 phenotype was determined by flow cytometry with HLA-A2 conjugated with APC (BB7.2, Biolegend) and HLA-A2 conjugated with FITC (A28, Miltenyi). 120 mL of peripheral blood was obtained from HLA-A2 negative donors and PBMCs were isolated using standard density gradient centrifugation. CD4+ cells were enriched by MACS technology prior to a cocktail of antibody staining: CD4 VioBlue (REAQL103), CD25 APC (REAL128), CD127 PE-Vio770 (REAL102), CD45RA FITC (REAL164), all from Miltenyi and CCR7 PE (G043H7, Biolegend). Stained cells were resuspended in Tyto buffer (Miltenyi). CD4+CD25highCD127lowCCR7+ Treg cells were sorted with a GMP-compatible Tyto sorter in a sterile environment with a normal speed Cartridge (Miltenyi). After two rounds of sorting, sorted cells were counted manually with Trypan blue (Gibco) in a hemacytometer under a light microscopy (Zeiss). 100,000 cells were seeded per well in a 96-well U plate (Corning) with 200 µL of Treg medium consisting of X-Vivo medium (Lonza), 10% FCS (Biochrom), 500 IU/mL recombinant human IL-2 (Miltenyi), and 10 uM rapamycin (Pfizer). For initial stimulation, 400,000 Treg expansion beads (Miltenyi) were added to each well (ratio of beads to cells is 4:1) on next morning. Medium was replenished every 2 to 3 days. Cells were counted on day 5 and were stimulated with Treg expansion beads at a ratio of 1:1 for another two days until electroporation.
*Screening of synthetic guide RNAs targeted CD3 epsilon locus:* In this study, five guide RNAs (gRNAs) were screened based on the criteria of CFD off-target < 5-10 calculated by CRISPR scan for Cas12a (Moreno-Mateos et al., 2015) and COSMID for Cas9 gRNA (Cradick et al., 2014). Among them, three gRNAs targeted exon 3 of CD3e, and two gRNAs targeted exon 6 of CD3e. These gRNAs were synthesized by IDT and their target sequence (SEQ 1 NO 9). Each gRNA was reconstituted in nuclease-free TE buffer to a stock concentration of 100 µM. Each gRNA formed Ribonucleoprotein (RNP) with cas12a and RNP was electroporated into conventional T cells (stimulated 2 days by plate-bound 1 µg/mL of αCD3/αCD28, more details see Kath et al17). Knock-out (KO) efficiency was checked on day 4 post electroporation, and cells were stained with CD3 PacBlue (UCHT1, Biolegend) and DAPI (Thermo Fisher) and were subsequently acquired on a CytoFLEX LX (Beckman Coulter). Mock cells were used for stain control.
*Donor template HDRT for generation of TRAC-CAR and CD3e-TruC:* Three donor templates were used in this study, one for integration in exon 1 of the TRAC locus and the other two for integration in exon 3 and exon 6 of the CD3e locus, respectively. Anti-HLA-A2 single chain variable fragment (scFv, clone 3PF12) with medium affinity (REF) was used and its mRNA sequence is available on Genebank (accession numbers AF163307 and AF163308). Each donor template was designed in Snapgene (Dotmatics) and synthesized by IDT as gBlocks gene fragments (double-stranded DNA fragments, 1600 - 2800 bp in length). gBlocks were cloned in plasmid PUC19 vector backbone using multiple fragment In-Fusion cloning according to the manufacturer's recommendation (Clontech, Takara). Plasmid transformation, colony PCR, and plasmid purification and validation were previously described (Kath et al., 2022). HA-flanked donor templates were amplified from the validated plasmids by PCR using the KAPA HiFi HotStart 2x Readymix (Roche) with reaction volumes of 500 µL. Primers used can be found in the sequence table (SEQ ID NO 43 to 46). PCR products were purified and concentrated using paramagnetic beads (AMPure XP, Beckman Coulter Genomics). The concentration of HDRT was determined by NanoDrop 1000 spectrophotometer (ThermoFisher) or Qubit (ThermoFisher) and was adjusted to a concentration of 1 µg/µL in nuclease-free water and stored at -20°C until use.
*Electroporation:* Upon 7 to 9 days of stimulation of Treg cells, beads were removed by a separator (MACSiMAGTM, Miltenyi) and bead-free Treg cells were counted and washed twice in PBS (Gibco) and 1 x106 cell pellets were resuspended in 20 µL of P3 buffer (Lonza). Electroporation was performed in a 16-well nucleocuvetteTM strip on a 4D-Nucleofector Device (Lonza) using the program EH-115. Formulation of RNP and mix with HDRT was previously described (Kath et al., 2022; Nguyen et al., 2020) 0.5 µg of HDRT was used per electroporation. To rescue cells, 90 µL of pre-warmed Treg medium was added to each well immediately after electroporation, incubated for 10 mins in a 37°C incubator prior to evenly transferring them to 2 wells of a 96-well U plate containing 150 µL pre-warmed Treg medium. On the next morning, cells were stimulated with Treg expansion beads at a ratio of 1:1.
*CASTseq for off-target identification:* Off-target analysis of gRNA was done as previously described (Turchiano et al., 2021). CD3e KO by using gRNA1 and mock Tconv cells from two biological replicates were generated and expanded for 2 weeks. Up to 10 million cells were washed in PBS, pelleted, and stored at -20°C for a short period of time. KO efficiencies on protein level were determined by flow cytometer prior to the cell pellet. Genomic DNAs were isolated using Quick-DNA Miniprep Plus Kit (Zymo Research).
*Expansion of CD3e-TruC and TRAC-CAR Treg cells:* Firstly, TRAC-CAR Treg cells were evenly distributed into 4 wells in a 96-well flat plate after electroporation. They were stimulated with 1) plate-bound 2 µg/mL HLA-A2 dimer:IgG fusion protein (BD) without or 2) with plate-bound 1 µg/mL αCD28 (Biolegend), or 3) gamma-irradiated (30 Gy) HLA-A2+ K562 cell line (a gift from Fatih Noyan, MHH) at a ratio of 1:1. Plate coating with antigen and/or antibody were prepared one day in advance and left in the fridge until use. No stimulation was included as a negative control. Untouched Treg cells stimulated with beads were included as a high control. Stimulation and medium replenishment were repeated every 2 to 3 days. Subsequently, we applied two approaches to expand CD3e-TruC and TRAC-CAR Treg cells: 1) expansion with Treg expansion beads every 2 to 3 days at a ratio of 1:1; 2) plate-bound 2 µg/mL HLA-A2 dimer:IgG fusion protein and 1 µg/mL αCD28. Cells were expanded two weeks after electroporation. Beads were removed and cells rest overnight prior to assay setup and readout.
*Gene editing efficiency checked by flow cytometry:* 100,000 to 200,000 Treg cells were cultured without beads overnight. TRAC-CAR Treg cells were stained with CD3 PB and αFc A647 (Jackson Immuno Research Labs). CD3e-TruC Treg cells were stained with CD3 PB and αMyc A647 (Cell Signaling). CD3e-TruC cells were also stained firstly with 5 µg/mL HLA-A2-Biotin (conjugated by lab managers from DRFZ), then stained with Steptavidin A555 (Invitrogen) and α Myc A647. Untouched wild-type Treg cells were used as control. Cells were resupsended in DAPI containing PBS prior to acquisition by CytoFLEX LX. Gene editing efficiency of Treg cells at d4 and d12 after electroporation was checked, separately.
*Purification of gene-edited Treg cells using Aria II sorter:* Expanded CD3e-TruC Treg cells and TRAC-CAR Treg cells were taken out of culture. Beads were removed prior to cell staining. CD3e-TruC Treg cells were sorted as NearIR-CD3+CD4+Myc+ cells and TRAC-CAR Treg cells were sorted as NearIR-CD3+CD4+αFc+ cells with an Aria II sorter using an 85 µm nozzle size (BD). Sorted cells were collected in a 15 mL Falcon tube (pre-coated with 5 mL FCS and then contained 3 mL Treg medium). Purity checks were performed after sorting. Sorted cells were centrifuged and resuspended in Treg medium at a concentration of 0.5 Mio cells per mL. Treg expansion beads were added in the culture at a ratio of 1:1 for expansion.
*Phenotypic characterization of gene-edited Treg cells:* On day 9 of expansion, 1 Mio of gene-edited and untouched Treg cells was taken out of culture, and beads were removed. Tconv cells were used as a negative control. One-third of cells were stained with fixable dye Aqua (Invitrogen) extracellularly, then cells were fixed and permeabilized using Transcription Factor Buffer Set (BD). αFc A647 for TRAC-CAR Treg cells and αMyc A647 for CD3e-TruC were stained intracellularly, followed by 2 times wash of permeabilization buffer. Cells were then stained by an antibody cocktail of CD3 PB, CD4 PE (Beckman Coulter), CD25 PC7 (Beckman Coulter), and FOXP3 FITC (BD). For cytokine profile examination of gene-edited Treg cells, another two third of cells were stimulated by 10 ng/mL PMA and 2.5 µg/mL lonomycin (Sigma Aldrich) for 6 hours. No stimulation was included as a negative control. Brefeldin A (Sigma Aldrich) was added at a concentration of 10 µg/mL after 1 h of co-culture. Upon 6h of stimulation, cells were harvested and stained similarly as above but used TNFα A700 (Biolegend), IFNγ APC-eF780 (Invitrogen), and IL-2 PE-Cy7 (Biolegend) instead of CD25 and FOXP3 antibodies. Stained cells were acquired at Cytoflex LX. 0.3 million to 1 million Treg cells were snap-freeze and stored at liquid nitrogen until genomic DNA isolation using established methods.
*Phosphorylation of ERK upon stimulation:* Beads were removed from the culture. Purified CD3e-TruC and TRAC-CAR as well as WT Treg cells were cultured in bead-free X-Vivo medium supplemented with 10% FCS and 1% Pen/Strep without IL-2 and rapamycin overnight prior to assay. Plate coating of either 5 µg/mL HLA-A2:IgG dimer (BD) or 10 µg/mL αCD3 antibody (Invitrogen) in a 48 well plate one day in advance in a 4°C fridge. The next day, cells were firstly harvested, counted, and stained with fixable live/dead dye UV. 0.5 Mio cells were seeded in each well on ice. Shortly spin down, then immediately incubated at 37°C on a thermomixer (Eppendorf). Upon indicated time period: 0 min, 2 min, 5 min, 10 min, 15 min, and 30 min, 200 µL pre-warmed 4% fixation buffer (Biolegend) was added and pipette gently and leave at 37°C for 15 min to stop stimulation. Cells were washed and supernatant was discarded. 200 µL of True-Phos perm buffer (pre-cooled at -20°C) were added to each well and incubated at -20°C for 60 min or longer. Cells were washed twice with PBS prior to intercellular stain with anti-ERK1/2 Phospho (Thr202/Tyr204) FITC (Biolegend) for 30 min at 4°C. Cells were washed twice with PBS and acquired at Cytoflex LX.
*Activation profiles of CD3e-TruC versus TRAC-CAR Treg cells:* Same as above, cells were rested overnight without IL-2 and rapamycin. Plate coating of HLA-A2:IgG dimer (BD) in a serial dilution of 10 - 5 - 2.5 - 1 µg/mL with or without 1 µg/mL αCD28 in a 96-well flat plate one day in advance in a 4°C fridge. CD3e-TruC and TRAC-CAR as well as WT Treg cells were resuspended in X-Vivo medium supplemented with 10% FCS and 100 IU/mL IL-2. 0.4 Mio cells were seeded in each well for stimulation for 1 day. No stimulation wells were included as a negative control. Tconv cells were included in parallel as a control. Upon 1 day of stimulation, cells were harvested and split into two wells for activation antibody staining in two panels. Examined antibodies here included: CD25 PC7 (Beckman), CD71 BV786 (BD bioscience), FOXP3 FITC (BD Pharmingen), ICOS (CD278) BV650, LAP PE, CTLA-4 (CD152) BV421, CD137 PE, CD154 BV421, Helios Percp-Cy5.5 (all from Biolegend). Among them, Helios and FOXP3 were stained intracellularly. Stained cells were immediately acquired at Cytoflex LX.
*Suppression assay:* Same as above, Treg cells were rested overnight without IL-2 and rapamycin. Autologous Tconv cells were enriched from PBMCs as responder cells (Tresp). Tresp cells were labeled with CFSE at a concentration of 1 µM for 10 mins at 37°C. Labeled cells were washed twice with 5 mL of FCS and rested at 37°C for 1 hour before seeding 50,000 cells per well. Treg cells were seeded at a range of ratio from 1:1 to 1:1/8 of Tresp: Treg. As stimuli, we used either Treg suppression inspector, human (Miltenyi) at a 1:1 ratio of total seeded cells per well to beads, or HLA-A2 positive donors' CD3-depleted PBMCs (A2+ cells), pre-labeled with CTFraRed and seeded at a 1:1 ratio of Tresp + Treg to A2+ cells, or together with beads and A2+ cells. Each condition has technical triplicates. Wells contains Tresp with either stimulus but not Treg cells as a positive control of proliferation. Wells contains Tresp alone as a negative control of proliferation. Cells were all resuspended in X-vivo medium supplemented with 10% FCS and 1% pen/streptavidin. Upon 5 days of incubation, cells were harvested and stained with CD4 PE (Beckman) and CD8 PE-Cy7 (BD Pharmingen) and finally resuspended in DAPI containing PBS for immediate acquisition at Cytoflex LX. % suppression capacity of Treg cells = (% divided Tresp alone - % divided Tresp treated with Treg)/ % divided Tresp alone *100.

### REFERENCES

1. Roemhild, A. et al. Regulatory T cells for minimising immune suppression in kidney transplantation: phase I/IIa clinical trial. BMJ 371, (2020).
2. Landwehr-Kenzel, S. et al. Adoptive transfer of ex vivo expanded regulatory T cells improves immune cell engraftment and therapy-refractory chronic GvHD. Mol Ther 30, 2298-2314 (2022).
3. Marek-Trzonkowska, N. et al. Therapy of type 1 diabetes with CD4(+)CD25(high)CD127-regulatory T cells prolongs survival of pancreatic islets - results of one year follow-up. Clin Immunol 153, 23-30 (2014).
4. Voskens, C. et al. Autologous regulatory T-cell transfer in refractory ulcerative colitis with concomitant primary sclerosing cholangitis. Gut gutjnl-2022-327075 (2022) doi:10.1136/gutjnl-2022-327075.
5. Amini, L. et al. Super-Treg: Toward a New Era of Adoptive Treg Therapy Enabled by Genetic Modifications. Frontiers in Immunology 11, 3868 (2021).
6. Landwehr-Kenzel, S. et al. Novel GMP-compatible protocol employing an allogeneic B cell bank for clonal expansion of allospecific natural regulatory T cells. Am J Transplant 14, 594-606 (2014).
7. Dawson, N. A. J. et al. Functional effects of chimeric antigen receptor co-receptor signaling domains in human regulatory T cells. Sci Transl Med 12, (2020).
8. Micklethwaite, K. P. et al. Investigation of product-derived lymphoma following infusion of piggyBac-modified CD19 chimeric antigen receptorT cells. Blood 138, 1391-1405 (2021).
9. Bishop, D. C. et al. Development of CAR T-cell lymphoma in 2 of 10 patients effectively treated with piggyBac-modified CD19 CAR T cells. Blood 138, 1504-1509 (2021).
10. Wilson, M. H. & Gottschalk, S. Expect the unexpected: piggyBac and lymphoma. Blood 138, 1379-1380 (2021).
11. Roth, T. L. et al. Reprogramming human T cell function and specificity with non-viral genome targeting. Nature 559, 405-409 (2018).
12. Schober, K. et al. Orthotopic replacement of T-cell receptor α- and β-chains with preservation of near-physiological T-cell function. Nat Biomed Eng 3, 974-984 (2019).
13. Muller, Y. D. et al. Precision Engineering of an Anti-HLA-A2 Chimeric Antigen Receptor in Regulatory T Cells for Transplant Immune Tolerance. Frontiers in Immunology 12, 3717 (2021).
14. Kath, J. et al. Pharmacological interventions enhance virus-free generation of TRAC-replaced CAR T cells. Mol Ther Methods Clin Dev 25, 311-330 (2022).
15. MacLeod, D. T. et al. Integration of a CD19 CAR into the TCR Alpha Chain Locus Streamlines Production of Allogeneic Gene-Edited CAR T Cells. Molecular Therapy 25, 949-961 (2017).
16. Eyquem, J. et al. Targeting a CAR to the TRAC locus with CRISPR/Cas9 enhances tumour rejection. Nature 543, 113-117 (2017).
17. Zhang, L. et al. AsCas12a ultra nuclease facilitates the rapid generation of therapeutic cell medicines. Nat Commun 12, 3908 (2021).
18. Moreno-Mateos, M. A. et al. CRISPRscan: designing highly efficient sgRNAs for CRISPR-Cas9 targeting in vivo. Nat. Methods 12, 982-988 (2015).
19. Cradick, T. J., Qiu, P., Lee, C. M., Fine, E. J. & Bao, G. COSMID: A Web-based Tool for Identifying and Validating CRISPR/Cas Off-target Sites. Mol Ther Nucleic Acids 3, e214 (2014).
20. Nguyen, D. N. et al. Polymer-stabilized Cas9 nanoparticles and modified repair templates increase genome editing efficiency. Nat Biotechnol 38, 44-49 (2020).
21. Turchiano, G. et al. Quantitative evaluation of chromosomal rearrangements in gene-edited human stem cells by CAST-Seq. Cell Stem Cell 28, 1136-1147.e5 (2021).
22. Baeuerle, P. A., et al. Synthetic TRuC receptors engaging the complete T cell receptor for potent anti-tumor response. Nature communications 10(1), 1-12 (2019).
23. Watkins, N. A. et al. The isolation and characterisation of human monoclonal HLA-A2 antibodies from an immune V gene phage display library. Tissue Antigens 55(3):219-28 (2000).

## Claims

1. A nucleic acid construct comprising:
- a first nucleic acid sequence region, encoding an antigen binding domain, and
- a second nucleic acid sequence region, comprising a targeting sequence configured for integrating the construct in-frame with an endogenous CD3 epsilon gene of a human cell.

2. The nucleic acid construct according to claim 1, wherein the targeting sequence is configured for integrating the construct in-frame into exon 3 or exon 6 of the endogenous CD3 epsilon gene, preferably wherein the target site of the CD3 epsilon gene, into which the first sequence region is integrated, is positioned downstream of a sequence encoding a CD3 epsilon signal peptide and upstream of an endogenous CD3 epsilon sequence.

3. The nucleic acid construct according to any of the preceding claims, wherein the targeting sequence of the second sequence region is configured for integration into a host genome by a gene editing technique, preferably CRISPR-Cas, zinc finger nucleases (ZFNs), integrases, site specific recombinases, meganucleases, homing endonucleases, or TALENs, more preferably a CRISPR-Cas12a derived from *Acidaminococcus species BV3L6* or CRISPR-Cas9 derived from Streptococcus pyogenes, and/or
wherein the targeting sequence comprises an identical sequence to a recognition site of the endogenous DNA sequence into which integration is intended, preferably selected from the group consisting of a homology arm, a guide RNA target site, a restriction enzyme recognition site, a ZFN recognition site, a ZFN cleaving site, a TALEN DNA-binding site, a recombinase recognition site, an integrase site and/or a homing nuclease recognition site.

4. The nucleic acid construct according to any of the preceding claims, wherein the antigen binding domain is in the form of an antibody or antigen-binding fragment thereof.

5. The nucleic acid construct according to any of the preceding claims, wherein the antigen binding domain is specific for an HLA protein, preferably HLA-A2.

6. A genetically modified human T cell, comprising an exogenous nucleic acid sequence region encoding an antigen binding domain integrated in-frame into an endogenous CD3 epsilon gene.

7. The genetically modified human T cell according to claim 6, wherein the genetically modified human T cell is a regulatory T cell (Treg).

8. The genetically modified T cell according to claim 6 or 7, wherein a CD3 epsilon protein is expressed as an in-frame fusion protein comprising the antigen binding domain and endogenous CD3 epsilon protein.

9. The genetically modified T cell according to any of claims 6 to 8, wherein T cell receptor (TCR) complex function is maintained in the presence of the in-frame fusion protein comprising the antigen binding domain and endogenous CD3 epsilon protein.

10. The genetically modified T cell according to any of claims 6 to 9, wherein the fusion protein is expressed under the control of an endogenous CD3 epsilon promoter.

11. The genetically modified T cell according to any of claims 6 to 10, wherein the exogenous nucleic acid sequence region encoding an antigen binding domain is integrated in-frame into exon 3 or exon 6 of the endogenous CD3 epsilon gene.

12. The genetically modified T cell according to any of claims 6 to 11, wherein the target site of the CD3 epsilon gene, into which the first sequence region is integrated, is positioned downstream of a sequence encoding a CD3 epsilon signal peptide and upstream of an endogenous CD3 epsilon sequence (generating an N-terminal fusion after removal of the signal peptide).

13. The genetically modified T cell according to any of claims 6 to 12, wherein the antigen binding domain binds an HLA protein, preferably HLA-A2.

14. The genetically modified T cell according to any of claims 6 to 13 for use as a medicament for the prevention and/or treatment of an unwanted (pathogenic) immune reaction, preferably an unwanted (pathogenic) immune reaction prior to and/or after an allogeneic transplantation or an autoimmune disease, more preferably for the prevention and/or treatment of graft-versus-host disease (GVHD).

15. A pharmaceutical composition comprising a genetically modified cell according to any of claims 6 to 13 and a pharmaceutically acceptable carrier.

16. A method for preparing a therapeutic genetically modified T cell, comprising repetitive stimulation of a genetically modified T cell according to any of claims 6 to 13 via the TCR/CD3 complex, wherein said therapeutic genetically modified T cell exhibits in essence unmodified expansion dynamics in culture (+/- 50%) in comparison to an unmodified T cell.

17. The method according to claim 16, wherein the genetically modified human T cell is a regulatory T cell (Treg).
